# EUROPEAN PATENT SPECIFICATION

(11) **EP 3 798 615 B1**
(45) Date of publication and mention of the grant of the patent: **20.12.2023**
(21) Application number: 19807646.5
(22) Date of filing: 21.05.2019
(51) Int. Cl.: G01N 21/76, G01N 33/53, G01N 33/58

(54) **CHEMILUMINESCENCE ANALYTICAL METHOD AND SYSTEM AND KIT USING SAME**
ANALYTISCHES CHEMILUMINESZENZVERFAHREN UND SYSTEM UND KIT MIT VERWENDUNG DAVON
PROCÉDÉ ET SYSTÈME ANALYTIQUES DE CHIMIOLUMINESCENCE ET KIT UTILISANT CEUX-CI

(30) Priority: 21.05.2018 CN 201810491290; 21.05.2018 CN 201810526541
(43) Date of publication of application: 31.03.2021
(73) Proprietor: Chemclin Diagnostics (Shanghai) Co., Ltd., Pudong, Shanghai 201210 (CN); Chemclin Diagnostics Co., Ltd., Beijing 100094 (CN)
(72) Inventor: YANG, Yang, Shanghai 201210 (CN); LIU, Yuhui, Beijing 100094 (CN); LI, Lin, Beijing 100094 (CN)
(74) Representative: Pharma Patents International AG
(86) International application number: PCT/CN2019/087828
(87) International publication number: WO 2019/223691

(56) References cited:
- CN-A- 101 326 440
- CN-A- 104 126 121
- CN-A- 104 969 069
- CN-A- 108 152 505
- CN-A- 109 470 860
- CN-U- 207 147 974
- NO-B- 176 071

## Description

### Field of the Invention

The present disclosure relates to the technical field of chemiluminescence, and in particular, to a chemiluminescence immune analytical method, a system using the chemiluminescence immune analytical method, and use of a kit.

### Background of the Invention

Chemiluminescence immune analysis is a non-radioactive immunodetection technique evolved rapidly in recent years. It uses chemiluminescent substance(s) to magnify a signal associated directly with the binding of an antibody to an antigen, thus to detect the binding process through the luminescent intensity of the chemiluminescent substance(s). Chemiluminescence immune analysis has become one of the most important techniques in the field of immunology detection. Light initiated chemiluminescent assay is a common technique applied in the field of chemiluminescence analysis. It can be used to study the interactions of biomolecules. Clinically, it is mostly utilized for the detection of diseases. Light initiated chemiluminescent assay utilizes and integrates the knowledge of many related fields especially macromolecular particles, organic synthesis, protein chemistry and clinical detection. Compared with traditional enzyme-linked immuno sorbent assay (ELISA), light initiated chemiluminescent assay is homogeneous, more sensitive, easier to operate and more automatic, and thus can found wide applications in the future.

In a double-antibody sandwich assay of the chemiluminescence immune analysis, when the concentration of an analyte reaches a certain value, no double-antibody sandwich complex can be formed, and a low signal value is therefore observed. This phenomenon is called high dose hook effect (HD-HOOK effect). In other words, HD-HOOK effect refers to a phenomenon where in a double-site sandwich immunological experiment, the linear orientation of a high dose section of a dose response curve does not rise indefinitely, but drops like a hook, resulting in false negatives. The HD-HOOK effect occurs frequently in immunodetection, and its occurrence rate accounts for 30% of positive samples. Due to the HD-HOOK effect, one cannot tell whether a concentration of a sample to be detected has exceeded the linear range of the detection kit or the concentration of the sample is really the detected value. The occurrence rate of false negatives is thus increased.

Therefore, there is an urgent need to provide a chemiluminescence immune analytical method which can broaden the detection range and avoid the HD-HOOK effect.

### Summary of the Invention

Directed against the defect in the existing technologies, the present disclosure aims to provide a chemiluminescence analytical method. The method of the present disclosure broadens a detection range and indicates an HD-HOOK sample or a sample that is beyond the detection range by way of selecting two signal values from signal values that are read in multiple times, and simply and quickly calculates the concentration of an analyte during detection.

In order to realize the above and associated objectives, the present disclosure provides, at a first aspect, a chemiluminescence immune analytical method, which includes the following steps of:
(1) mixing a sample to be detected suspected of containing a target molecule to be detected with a reagent required for a chemiluminescence reaction to react so as to form a mixture to be detected;
(2) initiating the mixture to be detected for t times successively to generate chemiluminescence, and recording a signal value of the chemiluminescence for n times, a signal value of the chemiluminescence recorded at an n^{th} time is marked as RLUn;
(3) selecting any two signal values from the n-time recorded signal values of the chemiluminescence, marking the two signal values respectively as RLUm and RLUk, and marking a growth rate from RLUm to RLUk as A;
(4) plotting a standard curve based on a growth rate A' from RLUm' to RLUk' in any two reactions at step (2) and step (3) with respect to a series of standard substances with known concentrations containing the target molecule to be detected; and
(5) comparing the growth rate A obtained at step (3) with the standard curve obtained at step (4) to determine a concentration of the target molecule to be detected,
t, n, m, and k are all natural numbers larger than 0, and k<m≤n≤t, n≥2.

In some embodiments of the present disclosure, the growth rate A=(RLUm/RLUk-1) × 100%; and/or, wherein n is larger than 2.

In some embodiments of the present disclosure, step (5) is: comparing the growth rate A obtained at step (3) with the standard curve obtained at step (4), when the growth rate A is located in a rising section of the standard curve, step (2) is stopped, and a concentration of the target molecule to be detected is calculated by directly putting RLUp read at a P^{th} time into the standard curve; and when the growth rate A is located in a dropping section of the standard curve, then step (2) is continued, and a concentration of the target molecule to be detected is calculated by putting RLUq of the reaction read at the q^{th} time into the standard curve,
p and q are both natural numbers larger than 0, and p≤q≤n;
preferably, wherein p is 1, and q is n.

In some preferred embodiments of the present disclosure, at step (1), the reagent required for the chemiluminescence reaction includes a acceptor reagent and a donor reagent,
the donor reagent includes a donor, and the donor is capable of generating singlet oxygen in an initiated state; and
the acceptor reagent includes a acceptor, and the acceptor is capable of reacting with the singlet oxygen so as to generate a detectable chemiluminescence signal value.

In some specific embodiments of the present disclosure, the method specifically includes the following steps of:
(a1) mixing a sample to be detected suspected of containing an antigen (or an antibody) to be detected with a acceptor reagent and performing a first incubation, and mixing a mixed liquid obtained from the first incubation with a donor reagent and performing a second incubation so as to form a mixture to be detected;
(a2) initiating the mixture to be detected for t times successively with irradiation of a red laser beam of 600-700 nm to generate chemiluminescence, and recording a signal value of the chemiluminescence for n times with a detection wavelength of 520-620 nm, a signal value of the chemiluminescence recorded at an n^{th} time is marked as RLUn;
(a3) selecting any two signal values from the n-time recorded signal values of the chemiluminescence, marking the two signal values respectively as RLUm and RLUk, and marking a growth rate from RLUm to RLUk as A, the growth rate A=(RLUm/RLUk-1) × 100%;
(a4) plotting a standard curve based on a growth rate A' from RLUm' to RLUk' in any two reactions at step (a2) and step (a3) with respect to a series of positive control samples with known concentrations containing the target molecule to be detected; and
(a5) comparing the growth rate A obtained at step (a3) with the standard curve obtained at step (a4) to determine a concentration of the target molecule to be detected,
t, n, m, and k are all natural numbers larger than 0, and k<m≤n≤t, n≥2.

In some embodiments of the present disclosure, step (a5) is: determining, based a value of A, whether the concentration of the target molecule to be detected is located in a rising section or a dropping section of the standard curve, and then calculating the concentration of the target molecule to be detected by putting RLUm of the target molecule to be detected into a corresponding standard curve.

In some other embodiments of the present disclosure, step (a5) is: comparing the growth rate A with the standard curve,
when the growth rate A is located in a rising section of the standard curve, step (a2) is stopped, and a concentration of the target molecule to be detected is calculated by directly putting RLUp read at a P^{th} time into the standard curve; and
when the growth rate A is located in a dropping section of the standard curve, the step (a2) is continued, and a concentration of the target molecule to be detected is calculated by putting RLUq of the reaction read at a q^{th} time into the standard curve,
p and q are both natural numbers larger than 0, and p≤q≤n.

The present disclosure provides, at a second aspect, a system using the chemiluminescence analytical method, which includes:
a reaction device, which is configured to conduct a chemiluminescence reaction therein;
an initiating and recording device, which is configured to initiate a mixture to be detected for t times successively to generate chemiluminescence, and record a signal value of the chemiluminescence for n times, a signal value of the chemiluminescence recorded at an n^{th} time is marked as RLUn; and which is configured to select any two signal values from n-time recorded signal values of the chemiluminescence, mark the two signal values respectively as RLUm and RLUk, and mark a growth rate from RLUm to RLUk as A; and
a processor, which is configured to plot a standard curve based on a growth rate A' from RLUm' to RLUk' in any two reactions with respect to a series of standard substances with known concentrations containing the target molecule to be detected; and which is configured to compare the growth rate A with the standard curve to determine a concentration of the target molecule to be detected,
t, n, m, and k are all natural numbers larger than 0, and k<m≤n≤t, n≥2.

In some specific embodiments of the present disclosure, a method of using the system includes the following steps of:
(1) mixing a sample to be detected suspected of containing a target molecule to be detected with a reagent required for a chemiluminescence immunoreaction to react so as to form a mixture to be detected;
(2) initiating the mixture to be detected for t times successively to generate chemiluminescence, and recording a signal value of the chemiluminescence for n times, a signal value of the chemiluminescence recorded at an n^{th} time is marked as RLUn;
(3) selecting any two signal values from n-time recorded signal values of the chemiluminescence, marking the two signal values respectively as RLUm and RLUk, and marking a growth rate from RLUm to RLUk as A;
(4) plotting a standard curve based on a growth rate A' from RLUm' to RLUk' in any two reactions at step (2) and step (3) with respect to a series of standard substances with known concentrations containing the target molecule to be detected; and
(5) comparing the growth rate A obtained at step (3) with the standard curve obtained at step (4) to determine a concentration of the target molecule to be detected,
t, n, m, and k are all natural numbers larger than 0, and k<m≤n≤t, n≥2.

In some embodiments of the present disclosure, the growth rate A=(RLUm/RLUk-1) × 100%; and/or, wherein n is larger than 2.

In some embodiments of the present disclosure, step (5) is: comparing the growth rate A obtained at step (3) with the standard curve obtained at step (4),
when the growth rate A is located in a rising section of the standard curve, step (2) is stopped, and a concentration of the target molecule to be detected is calculated by directly putting RLUp read at a P^{th} time into the standard curve; and
when the growth rate A is located in a dropping section of the standard curve, the step (2) is continued, and a concentration of the target molecule to be detected is calculated by putting RLUq of the reaction read at a q^{th} time into the standard curve,
t, n, m, k, p, and q are all natural numbers larger than 0, and k<m≤n≤t, p≤q≤ n, n≥2;
preferably, wherein p is 1, and q is n.

The present disclosure provides, at a third aspect, a use of a kit, which includes a reagent required for a chemiluminescence analysis in a method including the following steps of:
(1) mixing a sample to be detected suspected of containing a target molecule to be detected with a reagent required for a chemiluminescence reaction to react so as to form a mixture to be detected;
(2) initiating the mixture to be detected for t times successively to generate chemiluminescence, and recording a signal value of the chemiluminescence for n times, a signal value of the chemiluminescence recorded at an n^{th} time is marked as RLUn;
(3) selecting any two signal values from the n-time recorded signal values of the chemiluminescence, marking the two signal values respectively as RLUm and RLUk, and marking a growth rate from RLUm to RLUk as A;
(4) plotting a standard curve based on a growth rate A' from RLUm' to RLUk' in any two reactions at step (2) and step (3) with respect to a series of standard substances with known concentrations containing the target molecule to be detected; and
(5) comparing the growth rate A obtained at step (3) with the standard curve obtained at step (4) to determine a concentration of the target molecule to be detected,
t, n, m, and k are all natural numbers larger than 0, and k<m≤n≤t, n≥2.

In some specific embodiments of the present disclosure, the method of using the kit includes the following steps of:
(a1) mixing a sample to be detected suspected of containing an antigen (or an antibody) to be detected with a acceptor reagent and performing a first incubation, and mixing a mixed liquid obtained from the first incubation with a donor reagent and performing a second incubation so as to form a mixture to be detected;
(a2) initiating the mixture to be detected for t times successively with irradiation of a red laser beam of 600-700 nm to generate chemiluminescence, and recording a signal value of the chemiluminescence for n times with a detection wavelength of 520-620 nm, a signal value of the chemiluminescence recorded at an n^{th} time is marked as RLUn;
(a3) selecting any two signal values from n-time recorded signal values of the chemiluminescence, marking the two signal values respectively as RLUm and RLUk, and marking a growth rate from RLUm to RLUk as A, the growth rate A=(RLUm/RLUk-1) × 100%;
(a4) plotting a standard curve based on a growth rate A' from RLUm' to RLUk' in any two reactions at step (a2) and step (a3) with respect to a series of positive control samples with known concentrations containing the target molecule to be detected; and
(a5) comparing the growth rate A obtained at step (a3) with the standard curve obtained at step (a4) to determine a concentration of the target molecule to be detected,
t, n, m, and k are all natural numbers larger than 0, and k<m≤n≤t, n≥2.

In some embodiments of the present disclosure, step (a5) is: determining, based a value of A, whether the concentration of the target molecule to be detected is located in a rising section or a dropping section of the standard curve, and then calculating the concentration of the target molecule to be detected by putting RLUm of the target molecule to be detected into a corresponding standard curve.

In some other embodiments of the present disclosure, step (a5) is: comparing the growth rate A with the standard curve,
when the growth rate A is located in a rising section of the standard curve, step (a2) is stopped, and a concentration of the target molecule to be detected is calculated by directly putting RLUp read at a P^{th} time into the standard curve; and
when the growth rate A is located in a dropping section of the standard curve, the step (a2) is continued, and a concentration of the target molecule to be detected is calculated by putting RLUq of the reaction read at a q^{th} time into the standard curve,
p and q are both natural numbers larger than 0, and p≤q≤n.

The present disclosure has the following beneficial effects.
(1) The method of the present disclosure broadens a detection range and indicates an HD-HOOK sample or a sample that is beyond the detection range by way of selecting two signal values from signal values that are read in multiple times, and simply and quickly calculates the concentration of an analyte during detection.
(2) The method of the present disclosure is capable of 100% accurately determining an HD-HOOK-effect sample in a double-antibody sandwich assay, and therefore can distinctly improve accuracy of double-antibody sandwich immunoassays and reduce false negatives thereof.

### Brief Description of the Drawings

The present disclosure will be described in a more detailed way below with reference to the accompanying drawings.
Fig. 1 is a standard curve for a concentration and a corresponding signal value according to an embodiment of the present disclosure.
Fig. 2 is a standard curve for a concentration and a signal value growth rate A of multiple times of value reading.
Fig. 3 is a curve showing a relationship between a signal value and a sample concentration and a relationship between a growth rate A and the sample concentration in detection of HCG+β by a method of the present disclosure.
Fig. 4 is a curve showing a relationship between a signal value and a sample concentration and a relationship between a growth rate A and the sample concentration in detection of Ferr by a method of the present disclosure.
Fig. 5 is a curve showing a relationship between a signal value and a sample concentration and a relationship between a growth rate A and the sample concentration in detection of anti-HIV by a method of the present disclosure.
Fig. 6 is a curve showing a relationship between a signal value and a sample concentration and a relationship between a growth rate A and the sample concentration in detection of MYO by a method of the present disclosure.
Fig. 7 is a curve showing a relationship between a signal value and a sample concentration and a relationship between a growth rate A and the sample concentration in detection of NT-proBNP by a method of the present disclosure.
Fig. 8 is a curve showing a relationship between a signal value and a sample concentration and a relationship between a growth rate A and the sample concentration in detection of PCT by a method of the present disclosure.
Fig. 9 is a curve showing a relationship between a signal value and a sample concentration and a relationship between a growth rate A and the sample concentration in detection of cTnI by a method of the present disclosure.

### Detailed Description of the Embodiments

In order to make the present disclosure better understood, the present disclosure will be described in detail below. However, before describing the present disclosure in detail it shall be appreciated that the present disclosure is not limited to the following specific embodiments, and that the terms used herein are only for description of the specific embodiments, rather than limiting the protection scope of the present disclosure.

When a numerical value scope is provided, it shall be appreciated that, an upper limit and a lower limit of the scope and any intermediate value between any other specified or intermediate values are encompassed in the present disclosure. An upper limit and a lower limit of a smaller scope shall be independently included in the smaller scope, and is also encompassed in the present disclosure and in compliance with any limits expressly excluded from the specified scope. When the specified scope includes one or two limits, a scope excluding either or both of the limits is also included in the present disclosure.

Unless otherwise noted, the terms used herein all have same meanings as those understood by one of ordinary skill in the art. Although any methods or materials that are similar to or equivalent to those described in the present disclosure can also be used in implementation or assays of the present disclosure, preferred methods or materials are described.

Unless otherwise noted, the experiment method, detection method, and preparation method disclosed in the present disclosure all adopt commonly used techniques in the art, for example, commonly used techniques of molecular biology, biochemistry, chromatin structure and analysis, analytic chemistry, cell culturing, recombinant DNA technology, and other common techniques used in related fields. These techniques have been detailed in existing literature.

### I. Terms

The term "chemiluminescence immune analysis" used in the present disclosure is explained as follows. A chemical immune reaction can produce a product in an electronically initiated state, and when molecules of this product undergo a radiative transition or transfers energy to other molecules that emit light to cause the molecules to undergo a radiative transition, luminescence occurs. This phenomenon in which molecules are electronically initiated to emit light due to the absorption of chemical energy is called chemiluminescence. The method of using chemiluminescence for chemical immune analysis of the analyte is called a chemiluminescence immune analytical method.

The chemiluminescence immune analytical method not only can be a heterogeneous chemiluminescence immune analytical method but also can be a homogeneous chemiluminescence immune analytical method. The chemiluminescence immune analytical method can be a liquid-phase chemiluminescence analytical method, can be a gas-phase chemiluminescence analytical method, or can be a solid-phase chemiluminescence analytical method; and preferably, the chemiluminescence immune analytical method is a liquid-phase chemiluminescence analytical method. The chemiluminescence immune analytical method can be an ordinary chemiluminescence analytical method (an energy supplying reaction is an ordinary chemical reaction), can be a biochemiluminescence analytical method (an energy supplying reaction is a biochemical reaction, BCL for short), or can be an electrochemiluminescence analytical method (an energy supplying reaction is an electrochemical reaction, ECL for short); and preferably, the chemiluminescence immune analytical method is an ordinary chemiluminescence analytical method.

The term "target molecule to be detected" used in the present disclosure can be an immune molecule, such as an antigen or an antibody, can be an inorganic compound, such as a metal ion, hydrogen peroxide, CN⁻ or NO₂-, can be an organic compound, such as oxalic acid, ascorbic acid, imine, acetylcholine, etc., can be sugars, such as glucose or lactose, or can be amino acid, hormone, enzyme, fatty acid, vitamin and drug; and preferably, the term "target molecule to be detected" can be an immune molecule. The term "sample to be detected" used in the present disclosure contains target molecule to be detected. The term "mixed liquid to be detected" used in the present disclosure contains the sample to be detected.

The term "reagent required for a chemiluminescence immune analysis" used in the present disclosure refers to a reagent required in a chemical immune reaction to generate chemiluminescence. To generate chemiluminescence in a chemical immune reaction, the following requirements must be met: first, the reaction must provide enough initiation energy in a certain step only, because energy released in a prior step of the reaction disappears in the solution due to vibrational relaxation so that chemiluminescence does not occur; second, an advantageous reaction process is required, so that energy of the chemical immune reaction can be accepted by at least one substance and that an initiated state can be formed; and third, a molecule in the initiated state must have certain chemiluminescence quanta to effectively release photons or is capable of transferring its energy to another molecule so that another molecule enters the initiated state and releases photons.

The reagent required for a chemiluminescence immune analysis includes, but is not limited to the following substances: (1) a reactant in the chemiluminescence immune reaction; (2) a catalyst, a sensitizer, or an inhibitor in the chemiluminescence immune reaction; and (3) a reactant, a catalyst, a sensitizer etc. in a coupling reaction.

The "HOOK effect" used in the present disclosure refers to that in a double-antibody sandwich assay, when the concentration of an analyte reaches a certain value, no double-antibody sandwich complex can be formed, and a low signal value is therefore observed. In other words, the HOOK effect refers to a phenomenon where in a double-site sandwich immunological experiment, the linear orientation of a high dose section of a dose response curve does not rise indefinitely, but drops like a hook, resulting in false negatives.

The term "successively" used in the present disclosure is a time feature, and indicates that multiple times of "initiation" are distinguished by time units.

The term "antibody" used in the present disclosure is used in the broadest sense. The antibody includes any alloantibodies, and retains antibody fragments that can specifically bind to an antigen. The antibody includes, but is not limited to, Fab, Fv, scFv, and Fd fragments, chimeric antibodies, humanized antibodies, single-chain antibodies, bispecific antibodies, and fusion proteins containing the antigen-binding portion of the antibody and non-antibody proteins. If necessary, the antibody can be further conjugated with other moieties, such as biotin or streptavidin.

The term "antigen" used in the present disclosure refers to a substance with immunogenicity, such as protein and polypeptide. Representative antigens include (but are not limited to): cytokines, tumor markers, metalloproteins, cardiovascular diabetes related proteins, etc. The term "tumor marker" refers to a substance that is produced directly by tumor cells or by other cells of the body in response to the tumor cells during the development or proliferation of the tumor. It is indicative of the presence and growth of the tumor. Typical tumor markers in the art include (but not limited to): alpha fetoprotein (AFP), cancer antigen 125 (CA125), etc.

The term "bind" in the present disclosure refers to the direct combination between two molecules due to interactions such as covalent, electrostatic, hydrophobic, ionic and/or hydrogen bonding, including but not limited to interactions such as salt bridges and water bridges.

The term "specific binding" in the present disclosure refers to the mutual discrimination and selective binding reaction between two substances. From the perspective of the three-dimensional structure, it is the conformational correspondence between the corresponding reactants.

The term "biotin" in the present disclosure is widely present in animal and plant tissues. There are two ring structures on the molecule, namely an imidazolone ring and a thiophene ring. The imidazolone ring is the main part that binds to streptavidin. Activated biotin can be coupled with almost all known biomacromolecules, including proteins, nucleic acids, polysaccharides and lipids, etc., under the mediation of protein cross-linking agents. "Streptavidin" is a protein secreted by streptomyces, and has a molecular weight of 65kD. A "streptavidin" molecule is composed of 4 identical peptide chains, each of which can bind to a biotin. Therefore, each antigen or antibody can be coupled to multiple biotin molecules at the same time, thereby generating a "tentacle effect" to improve analysis sensitivity.

If necessary, any reagent used in the present disclosure, including the antigen, the antibody, the acceptor or the donor, can be conjugated with biotin or streptavidin according to actual needs.

The term "donor" in the present disclosure refers to a sensitizer that can produce a reactive intermediate, such as singlet oxygen, that reacts with the acceptor after being activated by energy or an active compound. The donor can be photoactivated (such as a dye and an aromatic compound) or chemically activated (such as an enzyme, a metal salt, etc.).

In some specific embodiments of the present disclosure, the donor is a photosensitizer. The photosensitizer may be a photosensitizer known in the art, preferably a compound that is relatively photo-stable and does not react effectively with singlet oxygen. Non-limiting examples include compounds such as methylene blue, rose bengal, porphyrin, phthalocyanine and chlorophyll disclosed in US Patent No. 5,709,994, and derivatives of these compounds having 1-50 atom substituents. The substituents are used to make these compounds more lipophilic or more hydrophilic, and/or serve as linking groups to members of a specific binding pair. Examples of other photosensitizers known to those skilled in the art can also be used in the present disclosure, such as those described in US Patent No. 6,406,913.

In some other specific embodiments of the present disclosure, the donor refers to other chemically activated sensitizers. Non-limiting examples are certain compounds that catalyze the conversion of hydrogen peroxide to singlet oxygen and water. Some other examples of the donor include: 1,4-dicarboxyethyl-1,4-naphthalene endoperoxide, 9,10-diphenylanthracene-9,10-endoperoxide, etc., and these compounds release singlet oxygen by heating or by directly absorbing light.

The term "acceptor" in the present disclosure refers to a compound that can react with singlet oxygen to generate a detectable signal. The donor is activated by energy or an active compound and releases singlet oxygen in a high-energy state. The singlet oxygen in a high-energy state is captured by a acceptor in a short distance to transfer energy so as to activate the acceptor.

In some specific embodiments of the present disclosure, the acceptor is a substance that undergoes a chemical reaction with singlet oxygen to form an unstable metastable intermediate, which can be decomposed and emit light at the same time or later on. Typical examples of the substance include, but are not limited to: enol ether, enamine, 9-alkylidene xanthan gum, 9-alkylidene-N-alkylacridans, aryl vinyl ether, diepoxyethylene, dimethyl thiophene, aromatic imidazole or lucigenin.

In some other specific embodiments of the present disclosure, the acceptor is an alkene capable of reacting with singlet oxygen to form hydroperoxides or dioxetanes that can be decomposed into ketones or carboxylic acid derivatives, can be stable dioxetanes decomposed by the action of light, can be acetylenes that can react with singlet oxygen to form diketones, can be hydrazones or hydrazides that can form azo compounds or azocarbonyl compounds, such as luminol, and can be aromatic compounds that can form endoperoxides. Specific non-limiting examples of the acceptor that can be utilized in accordance with the present disclosure and the claimed invention are described in US Patent No. 5,340,716.

In some other specific embodiments of the present disclosure, the "donor" and/or "acceptor" may be coated on the substrate by a functional group to form "donor microspheres" and/or "acceptor microspheres". The "substrate" in the present disclosure is a microsphere or particle known to those skilled in the art. The substrate can be of any size, can be organic or inorganic, can be expandable or non-expandable, and can be porous or non-porous. The substrate has any density, but preferably has a density close to that of water, preferably can float in water, and is composed of transparent, partially transparent or opaque materials. The substrate can be charged or not, and when charged, it is preferably negatively charged. The substrate can be solid (such as polymers, metals, glass, organic and inorganic substances such as minerals, salts and diatoms), small oil droplets (such as hydrocarbons, fluorocarbons, siliceous fluids), and vesicles (such as synthetic ones, such as phospholipids, or natural ones, such as cells, and cell organelles). The substrate can be latex particles or other particles containing organic or inorganic polymers, lipid bilayers such as liposomes, phospholipid vesicles, small oil droplets, silicon particles, metal sols, cells and microcrystalline dyes. The substrate is generally versatile or capable of binding to the donor or acceptor through specific or non-specific covalent or non-covalent interactions. There are many functional groups available or incorporated. Typical functional groups include carboxylic acid, acetaldehyde, amino, cyano, vinyl, hydroxyl, sulfhydryl and so on. A non-limiting example of the substrate suitable for use in the present disclosure is carboxyl modified latex particles. The details of this substrate can be found in US Patent No. 5,709,994 and No. 5,780,646.

### II. Specific embodiments

### Basic principles of double-antibody sandwich assay:

Basic principles of double-antibody sandwich assay are well-known to those skilled in the art. A conventional process of a double-antibody sandwich assay is as follows. A primary antibody is bound to a solid-phase carrier. The primary antibody is enabled to react first with an antigen and then with a labeled second antibody. A signal is finally detected by way of chemiluminescence reaction or enzyme-linked immune sorbent assay.

The present disclosure will be described in detail.

The present disclosure provides, at a first aspect, an immune analysis method, which includes the following steps of:
(1) mixing a sample to be detected suspected of containing a target molecule to be detected with a reagent required for a chemiluminescence reaction to react so as to form a mixture to be detected;
(2) initiating the mixture to be detected for t times successively to generate chemiluminescence, and recording a signal value of the chemiluminescence for n times, a signal value of the chemiluminescence recorded at an n^{th} time is marked as RLUn;
(3) selecting any two signal values from n-time recorded signal values of the chemiluminescence, marking the two signal values respectively as RLUm and RLUk, and marking a growth rate from RLUm to RLUk as A;
(4) plotting a standard curve based on a growth rate A' from RLUm' to RLUk' in any two reactions at step (2) and step (3) with respect to a series of standard substances with known concentrations containing the target molecule to be detected; and
(5) comparing the growth rate A obtained at step (3) with the standard curve obtained at step (4) to determine a concentration of the target molecule to be detected,
t, n, m, and k are all natural numbers larger than 0, and k<m≤n≤t, n≥2.

In some embodiments of the present disclosure, the growth rate A=(RLUm/RLUk-1) × 100%.

In some other embodiments of the present disclosure, n is larger than 2. For example, n can be 3, 4 or 5 and so on. In the present disclosure, when n is larger than 2, the detection of the method has relatively high sensitivity and strong capability of resisting the HD-HOOK effect.

In some embodiments of the present disclosure, step (5) is: comparing the growth rate A obtained at step (3) with the standard curve obtained at step (4),
when the growth rate A is located in a rising section of the standard curve, step (2) is stopped, and a concentration of the target molecule to be detected is calculated by directly putting RLUp read at a P^{th} time into the standard curve; and
when the growth rate A is located in a dropping section of the standard curve, the step (2) is continued, and a concentration of the target molecule to be detected is calculated by putting RLUq of the reaction read at a q^{th} time into the standard curve,
p and q are both natural numbers larger than 0, and p≤q≤n;
preferably,. wherein p is 1, and q is n.

In some preferred embodiments of the present disclosure, at step (1), the reagent required for the chemiluminescence reaction includes a acceptor reagent and a donor reagent,
the donor reagent includes a donor, and the donor is capable of generating singlet oxygen in an initiated state; and
the acceptor reagent includes a acceptor, and the acceptor is capable of reacting with the singlet oxygen so as to generate a detectable chemiluminescence signal value.

In some specific embodiments of the present disclosure, the method specifically includes the following steps of:
(a1) mixing a sample to be detected suspected of containing an antigen (or an antibody) to be detected with a acceptor reagent and performing a first incubation, and mixing a mixed liquid obtained from the first incubation with a donor reagent and performing a second incubation so as to form a mixture to be detected;
(a2) initiating the mixture to be detected for t times successively with irradiation of a red laser beam of 600-700 nm to generate chemiluminescence, and recording a signal value of the chemiluminescence for n times with a detection wavelength of 520-620 nm, a signal value of the chemiluminescence recorded at an n^{th} time is marked as RLUn;
(a3) selecting any two signal values from n-time recorded signal values of the chemiluminescence, marking the two signal values respectively as RLUm and RLUk, and marking a growth rate from RLUm to RLUk as A, the growth rate A=(RLUm/RLUk-1) × 100%;
(a4) plotting a standard curve based on a growth rate A' from RLUm' to RLUk' in any two reactions at step (a2) and step (a3) with respect to a series of positive control samples with known concentrations containing the target molecule to be detected; and
(a5) comparing the growth rate A obtained at step (a3) with the standard curve obtained at step (a4) to determine a concentration of the target molecule to be detected,
t, n, m, and k are all natural numbers larger than 0, and k<m≤n≤t, n≥2.

In some embodiments of the present disclosure, step (a5) is: determining, based a value of A, whether the concentration of the target molecule to be detected is located in a rising section or a dropping section of the standard curve, and then calculating the concentration of the target molecule to be detected by putting RLUm of the target molecule to be detected into a corresponding standard curve.

In some other embodiments of the present disclosure, step (a5) is: comparing the growth rate A with the standard curve,
when the growth rate A is located in a rising section of the standard curve, step (a2) is stopped, and a concentration of the target molecule to be detected is calculated by directly putting RLUp read at a P^{th} time into the standard curve; and
when the growth rate A is located in a dropping section of the standard curve, the step (a2) is continued, and a concentration of the target molecule to be detected is calculated by putting RLUq of the reaction read at a q^{th} time into the standard curve,
p and q are both natural numbers larger than 0, and p≤q≤n.

The present disclosure provides, at a second aspect, a system using the chemiluminescence analytical method, which includes:
a reaction device, which is configured to conduct a chemiluminescence reaction therein;
an initiating and recording device, which is configured to initiate a mixture to be detected for t times successively to generate chemiluminescence, and record a signal value of the chemiluminescence for n times, a signal value of the chemiluminescence recorded at an n^{th} time is marked as RLUn; and which is configured to select any two signal values from n-time recorded signal values of the chemiluminescence, mark the two signal values respectively as RLUm and RLUk, and mark a growth rate from RLUm to RLUk as A; and
a processor, which is configured to plot a standard curve based on a growth rate A' from RLUm' to RLUk' in any two reactions with respect to a series of standard substances with known concentrations containing the target molecule to be detected; and which is configured to compare the growth rate A with the standard curve to determine a concentration of the target molecule to be detected,
t, n, m, and k are all natural numbers larger than 0, and k<m≤n≤t, n≥2.

In some specific embodiments of the present disclosure, a method of using the system includes the following steps of:
(1) mixing a sample to be detected suspected of containing a target molecule to be detected with a reagent required for a chemiluminescence immunoreaction to react so as to form a mixture to be detected;
(2) initiating the mixture to be detected for t times successively to generate chemiluminescence, and recording a signal value of the chemiluminescence for n times, a signal value of the chemiluminescence recorded at an n^{th} time is marked as RLUn;
(3) selecting any two signal values from n-time recorded signal values of the chemiluminescence, marking the two signal values respectively as RLUm and RLUk, and marking a growth rate from RLUm to RLUk as A;
(4) plotting a standard curve based on a growth rate A' from RLUm' to RLUk' in any two reactions at step (2) and step (3) with respect to a series of standard substances with known concentrations containing the target molecule to be detected; and
(5) comparing the growth rate A obtained at step (3) with the standard curve obtained at step (4) to determine a concentration of the target molecule to be detected,
t, n, m, and k are all natural numbers larger than 0, and k<m≤n≤t, n≥2.

In some embodiments of the present disclosure, the growth rate A=(RLUm/RLUk-1) × 100%.

In some other embodiments of the present disclosure, n is larger than 2. For example, n can be 3, 4 or 5 and so on. In the present disclosure, when n is larger than 2, the detection of the system has relatively high sensitivity and strong capability of resisting the HD-HOOK effect.

In some embodiments of the present disclosure, step (5) is: comparing the growth rate A obtained at step (3) with the standard curve obtained at step (4),
when the growth rate A is located in a rising section of the standard curve, step (2) is stopped, and a concentration of the target molecule to be detected is calculated by directly putting RLUp read at a P^{th} time into the standard curve; and
when the growth rate A is located in a dropping section of the standard curve, the step (2) is continued, and a concentration of the target molecule to be detected is calculated by putting RLUq of the reaction read at a q^{th} time into the standard curve,
t, n, m, k, p, and q are all natural numbers larger than 0, and k<m≤n≤t, p≤q≤ n, n≥2;
preferably,.wherein p is 1, and q is n.

The present disclosure provides, at a third aspect, a kit, which includes a reagent required for a chemiluminescence analysis, and a method of using the kit includes the following steps of:
(1) mixing a sample to be detected suspected of containing a target molecule to be detected with a reagent required for a chemiluminescence reaction to react so as to form a mixture to be detected;
(2) initiating the mixture to be detected for t times successively to generate chemiluminescence, and recording a signal value of the chemiluminescence for n times, wherein a signal value of the chemiluminescence recorded at an n^{th} time is marked as RLUn;
(3) selecting any two signal values from n-time recorded signal values of the chemiluminescence, marking the two signal values respectively as RLUm and RLUk, and marking a growth rate from RLUm to RLUk as A;
(4) plotting a standard curve based on a growth rate A' from RLUm' to RLUk' in any two reactions at step (2) and step (3) with respect to a series of standard substances with known concentrations containing the target molecule to be detected; and
(5) comparing the growth rate A obtained at step (3) with the standard curve obtained at step (4) to determine a concentration of the target molecule to be detected,
t, n, m, and k are all natural numbers larger than 0, and k<m≤n≤t, n≥2.

In some embodiments of the present disclosure, at step (1), the chemiluminescence reaction is a homogeneous chemiluminescence reaction.

In some preferred embodiments of the present disclosure, at step (1), the reagent required for the chemiluminescence reaction includes a acceptor reagent and a donor reagent,
the donor reagent includes a donor, and the donor is capable of generating singlet oxygen in an initiated state; and
the acceptor reagent includes a acceptor, and the acceptor is capable of reacting with the singlet oxygen so as to generate a detectable chemiluminescence signal value.

In some embodiments of the present disclosure, the acceptor refers to macromolecular particles that are filled with a light-emitting compound and a lanthanide compound.

In some preferred embodiments of the present disclosure, the light-emitting compound is selected from an olefin compound, preferably selected from dimethyl thiophene, a dibutanedione compound, dioxene, enol ether, enamine, 9-alkylene xanthane, 9-alkylene-N-9,10 dihydroacridine, aryl etherene, aryl imidazole and lucigenin and their derivatives, more preferably selected from dimethyl thiophene and its derivatives.

In some other preferred embodiments of the present disclosure, the lanthanide compound is a europium complex.

In some embodiments of the present disclosure, the acceptor includes an olefin compound and a metal chelate, is in the form of unparticle, and is soluble in aqueous media.

In some specific embodiments of the present disclosure, the acceptor is bonded to a first specific conjugate of the target molecule to be detected directly or indirectly.

In some embodiments of the present disclosure, the donor refers to macromolecular particles that are filled with a light-sensitive compound, and is capable of generating singlet oxygen in response to irradiation of a red laser beam.

In some preferred embodiments of the present disclosure, the light-sensitive compound is selected from one of methylene blue, rose bengal, porphyrin, and phthalocyanine.

In some embodiments of the present disclosure, the donor is bonded to a label directly or indirectly.

In some preferred embodiments of the present disclosure, at step (1), the reagent required for the chemiluminescence reaction further includes a reagent of a second specific conjugate of the target molecule to be detected; and preferably, the second specific conjugate of the target molecule to be detected is bonded to a specific conjugate of a label directly or indirectly.

In some embodiments of the present disclosure, at step (1), the sample to be detected containing the target molecule to be detected is first mixed with a acceptor reagent and the reagent of the second specific conjugate of the target molecule to be detected, and a resulting mixture is then mixed with a donor reagent.

In some specific embodiments of the present disclosure, at step (2), the mixture to be detected is initiated by energy and/or an active compound so as to generate chemiluminescence; and preferably, the mixture to be detected is initiated by irradiation of a red laser beam of 600-700 nm to generate chemiluminescence.

In some other specific embodiments of the present disclosure, at step (2), a detection wavelength for recording the signal value of the chemiluminescence is 520-620 nm.

In some embodiments of the present disclosure, the target molecule to be detected is an antigen or an antibody. The antigen refers to an immunogenic substance, and the antibody refers to an immunoglobulin that is produced by an organism and is capable of recognizing a unique foreign substance.

In some other embodiments of the present disclosure, the standard substance is used as a positive control.

In some specific embodiments of the present disclosure, the method of using the kit includes the following steps of:
(a1) mixing a sample to be detected suspected of containing an antigen (or an antibody) to be detected with a acceptor reagent and performing a first incubation, and mixing a mixed liquid obtained from the first incubation with a donor reagent and performing a second incubation so as to form a mixture to be detected;
(a2) initiating the mixture to be detected for t times successively with irradiation of a red laser beam of 600-700 nm to generate chemiluminescence, and recording a signal value of the chemiluminescence for n times with a detection wavelength of 520-620 nm, wherein a signal value of the chemiluminescence recorded at an n^{th} time is marked as RLUn;
(a3) selecting any two signal values from n-time recorded signal values of the chemiluminescence, marking the two signal values respectively as RLUm and RLUk, and marking a growth rate from RLUm to RLUk as A, the growth rate A=(RLUm/RLUk-1) × 100%;
(a4) plotting a standard curve based on a growth rate A' from RLUm' to RLUk' in any two reactions at step (a2) and step (a3) with respect to a series of positive control samples with known concentrations containing the target molecule to be detected; and
(a5) comparing the growth rate A obtained at step (a3) with the standard curve obtained at step (a4) to determine a concentration of the target molecule to be detected,
t, n, m, and k are all natural numbers larger than 0, and k<m≤n≤t, n≥2.

In some embodiments of the present disclosure, step (a5) is: determining, based a value of A, whether the concentration of the target molecule to be detected is located in a rising section or a dropping section of the standard curve, and then calculating the concentration of the target molecule to be detected by putting RLUm of the target molecule to be detected into a corresponding standard curve.

In some other embodiments of the present disclosure, step (a5) is: comparing the growth rate A with the standard curve,
when the growth rate A is located in a rising section of the standard curve, step (a2) is stopped, and a concentration of the target molecule to be detected is calculated by directly putting RLUp read at a P^{th} time into the standard curve; and
when the growth rate A is located in a dropping section of the standard curve, the step (a2) is continued, and a concentration of the target molecule to be detected is calculated by putting RLUq of the reaction read at a q^{th} time into the standard curve,
p and q are both natural numbers larger than 0, and p≤q≤n.

It shall be particularly noted that the above method are not for diagnosis of diseases, but for broadening a detection range and indicating an HD-HOOK sample or a sample that is beyond the detection range by way of selecting two signal values from signal values that are read in multiple times during a double-antibody sandwich immunoassay or double-antigen sandwich immunoassay.

Preferably, the antigen refers to an immunogenic substance such as proteins and polypeptides. Typical antigens include (but not limited to): cytokines, tumor makers, metalloproteins, cardiovascular disease and glycuresis related proteins.

The antigen refers to an immunoglobulin that is produced by an organism and is capable of recognizing a unique foreign substance.

In the embodiments of the present disclosure, the antigen or antibody is selected from alpha fetoprotein (AFP), hepatitis B surface antibody (HBsAb), human chorionic gonadotropin and β subunit (HCG+β), hepatitis B surface antigen (HBsAg), cancer antigen 125 (CA125), C-peptide (CP), ferritin (Ferr), Anti-HCV and so on.

Samples that can be detected by the method of the present disclosure are not limited herein. They can be any samples containing a target antigen (or antibody) to be detected. Typical examples of such samples include serum samples, urine samples, saliva samples, etc. Preferred samples used in the present disclosure are serum samples.

Preferably, the label and the specific conjugate of the label can specifically bind to each other.

More preferably, the label is biotin, and the specific conjugate of the label is streptavidin.

Preferably, the acceptor refers to macromolecular particles that are filled with a light-emitting compound and a lanthanide compound. The light-emitting compound may be a derivative of dioxene or thioxene, and the lanthanide compound may be Eu(TTA)₃/TOPO or Eu(TTA)₃/Phen. The particles are available on the market. A surface functional group of the acceptor may be any group that can bind to a protein. Examples are various known functional groups such as carboxyl group, aldehyde group, amidogen, epoxy ethyl, or halogen alkyl that can bind to a protein.

Preferably, the donor refers to macromolecular particles that are filled with a light-sensitive compound. The donor is capable of generating singlet oxygen ions in response to the irradiation of a red laser beam. When donor is close enough to the acceptor, the singlet oxygen ions travel to the acceptor and react with the light-emitting compound within the acceptor, and then the light-emitting compound emits ultraviolet light. The ultraviolet light then excites the lanthanide compound which then emits photons having a certain wavelength. The light-sensitive compound may be phthalocyanine and is available on the market.

In a detection range, the concentration of the target antigen to be detected is reflected by the number of the double-antibody sandwich complex and is in direct proportion to the number of the photons. However, when the concentration of the target antigen to be detected is too high, some of the antigens to be detected respectively bind to a single antibody, as a consequence of which less double-antibody sandwich complexes are formed. This leads to a low light signal which cannot reflect the real concentration of the target antigen to be detected.

Similarly, in the detection range, the concentration of the target antibody to be detected is reflected by the number of the double-antigen sandwich complex and is in direct proportion to the number of the photons. However, when the concentration of the target antibody to be detected is too high, some of the antibodies to be detected respectively bind to a single antigen, as a consequence of which less double-antigen sandwich complexes are formed. This leads to a low light signal which cannot reflect the real concentration of the target antibody to be detected.

The above method broadens a detection range and indicates an HD-HOOK sample or a sample that is beyond the detection range by way of selecting two signal values from signal values that are read in multiple times. A difference between the two signal values is influenced by the following three aspects.

First, during a first time of value reading, singlet oxygen ions are released by the donor in response to the irradiation of the red laser beam (600-700 nm). Some of the singlet oxygen ions travel to the acceptor and emit, after a series of chemical reactions, high energy-level light of 520-620 nm. Other singlet oxygen ions react with the target antigen (or antibody) to be detected that is not bound by the antibody (or antigen), thereby reducing the concentration of the target antigen (or antibody) to be detected. For a low-concentration sample, after the concentration of the target antigen (or antibody) to be detected is reduced, less double-antibody sandwich complexes are formed, and therefore a signal value obtained at a second time of value reading is smaller. For a high-concentration sample, after the concentration of the target antigen (or antibody) to be detected is reduced, more double-antibody sandwich complexes are formed, and therefore a signal value obtained at the second time of value reading is larger.

Second, for a low-concentration sample, during the first time of value reading, the donor releases singlet oxygen ions when irradiated by the red laser beam (600-700 nm) and some energy thereof is consumed. That is why the signal value obtained at the second time of value reading is smaller.

Third, for an HD-HOOK sample, during the first time of value reading, the antigen-antibody reaction does not reach equilibrium, and during an interval between the two times of value reading, the reaction keeps proceeding in a forward direction. That is why the signal value obtained at the second time of value reading is larger.

To summarize, in the present disclosure, when the reaction does not reach equilibrium, the first time of value reading is performed. Singlet oxygen ions are released by the donor in response to the irradiation of the red laser beam. Some of the singlet oxygen ions travel to the acceptor, and other singlet oxygen ions react with the target antigen (or antibody) to be detected that is not bound so that part of the target antigen (or antibody) to be detected is consumed. In this way, the equilibrium of the reaction is shifted in a reverse direction. On the other hand, when the donor undergoes an irradiation, the energy thereof is consumed, and therefore for a low-concentration sample, the signal value with respect to the target antigen (or antibody) to be detected obtained at the second time of value reading is smaller. For a high-concentration sample, during the first time of value reading, the binding between the double-antibody sandwich complexes and the donor is far from equilibrium, and during the second time of value reading, the reaction shifts in the forward direction, and therefore the signal value is larger.

### III. Examples

In order to make the present disclosure better understood, the present disclosure is further detailed with reference to examples. These examples are illustrative only, and do not limit the application range of the present disclosure. Unless otherwise noted, raw materials or components used in the present disclosure are all available on the market or can be formed by conventional methods.

### Example 1: Detection of an alpha fetoprotein (AFP) sample by a conventional method and by a method of the present disclosure respectively

In the present example, an alpha fetoprotein (AFP) detection kit (chemiluminescence) produced by Shanghai Beyond Biotech Co., Ltd was used to measure a content of alpha fetoprotein in a sample.

Gradient dilution was performed on high-concentration antigens of alpha fetoprotein. Signal values of samples having different concentrations of alpha fetoprotein were detected by a conventional method and by a method of the present disclosure, respectively.

Detection by the conventional method includes the following steps:
An analyte sample with a known concentration (a known standard substance) was mixed with a reagent 1 (a solution of a acceptor bonded to a mouse monoclonal antibody) and a reagent 2 (a solution of a mouse monoclonal antibody bonded to biotin), and a resulting mixture was incubated at 37°C for 15 min. Then, a reagent 3 (a solution of a donor bonded to streptavidin) was added, and a resulting mixture was incubated at 37°C for 15 min to obtain a reaction solution. Laser irradiation was performed to the reaction solution, and a photon counter was used to read a signal value which was marked as RLU. Results were shown in Table 2.

Detection by performing multiple times of value reading according to the method of the present disclosure includes the following steps:
An analyte sample with a known concentration (a known standard substance) was mixed with a reagent 1 (a solution of a acceptor bonded to a mouse monoclonal antibody) and a reagent 2 (a solution of a mouse monoclonal antibody bonded to biotin), and a resulting mixture was incubated at 37°C for 15 min. Then, a reagent 3 (a solution of a donor bonded to streptavidin) was added, and a resulting mixture was incubated at 37°C for 1 min. A signal value RLU1 (1 min) was read. After that, the mixture was incubated again at 37°C, and when a time length of two times of incubation accumulates to 5 min, a signal value RLU2 (5 min) was read. Then, the mixture was incubated again at 37°C, and when a time length of three times of incubation accumulates to 10 min, a signal value RLU3 (10 min) was read. After that, the mixture was incubated again at 37°C, and when a time length of fourth times of incubation accumulates to 20 min, a signal value RLU4 (20 min) was read. Two of signal values read were selected to calculate a growth rate of the two signal values based on an equation A=(RLUm/RLUk-1)× 100%, k<m≤n. Detection results were shown in Table 1.

**Table 1:**

| AFP concentrations | Reading time min | | | | Growth rates of two signal values | | | |
|---|---|---|---|---|---|---|---|---|
| ng/mL | RLU1 (1min) | RLU2 (5min) | RLU3 (10min) | RLU4 (20min) | (RLU2/RLU1-1) × 100% | (RLU3/RLU1-1) × 100% | (RLU4/RLU1-1) × 100% | (RLU4/RLU2-1) × 100% |
| 50 | 2219 | 1560 | 1217 | 1057 | -29.69% | -45.17% | -52.37% | -32.25% |
| 200 | 6014 | 4453 | 3420 | 2945 | -25.95% | -43.14% | -51.03% | -33.86% |
| 800 | 26273 | 25056 | 20516 | 17906 | -4.63% | -21.91% | -31.85% | -28.53% |
| 3200 | 116831 | 155320 | 144980 | 135291 | 32.94% | 24.09% | 15.80% | -12.90% |
| 12800 | 391561 | 581432 | 579317 | 567994 | 48.49% | 47.95% | 45.06% | -2.31% |
| 51200 | 688438 | 1079654 | 1117775 | 1160253 | 56.83% | 62.36% | 68.53% | 7.47% |
| 204800 | 474318 | 773523 | 819849 | 832642 | 63.08% | 72.85% | 75.55% | 7.64% |
| 819200 | 142500 | 238776 | 253665 | 260325 | 67.56% | 78.01% | 82.68% | 9.02% |
| 3276800 | 24940 | 42648 | 45614 | 46874 | 71.00% | 82.89% | 87.95% | 9.91% |

As can be seen from Table 1, when the concentration is in a range from 50 ng/ml to 51,200 ng/ml, the signal value increases with the increase of the concentration; and when the concentration continues to increase, the signal value decreases with the increase of the concentration of alpha fetoprotein. That is, when the concentration is larger than 51,200 ng/ml, the HD-HOOK effect occurs. In a conventional detection, for a sample with an antigen concentration higher than this detection range, a detected concentration will be low (detected concentrations are all less than 51,200 ng/ml).

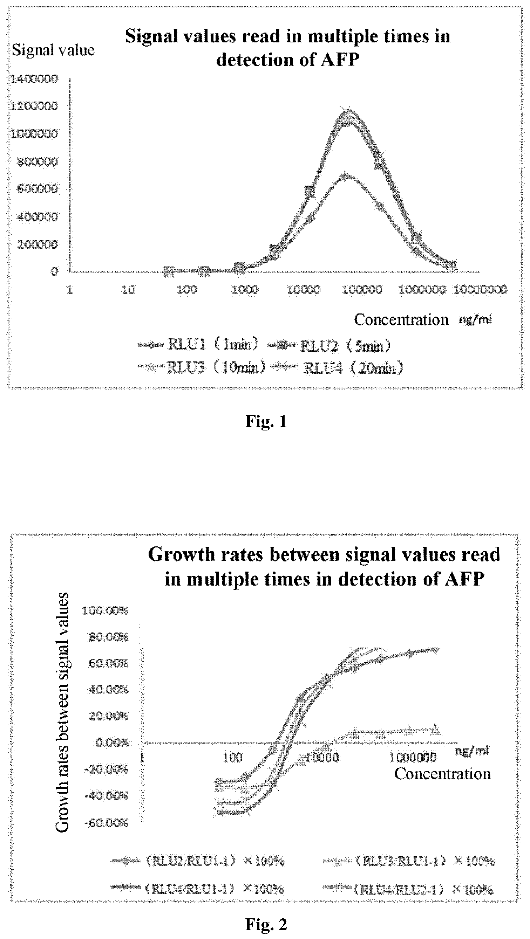

The method of the present disclosure broadens a detection range and indicates an HD-HOOK sample or a sample that is beyond the detection range by way of selecting two signal values from signal values that are read in multiple times.. Results of signal values are obtained after a series of detection to samples with known concentrations, and a standard curve between the concentrations and corresponding signal values and a standard curve between the concentrations and growth rates A are plotted (as shown in Fig. 1 and Fig. 2 respectively).

RLU2 (5 min) is selected, and a growth rate A of RLU4 (20 min) and RLU1 (1 min) of the sample to be detected obtained by performing two times of value reading is calculated based on an equation A=(RLU4/RLU1-1) × 100% as an index for determining a concentration section of the sample. As can be seen from Table 1 and Fig. 1, the signal value increases with the increase of the concentration before the concentration increases to 51,200 ng/ml (a rising section is defined); and then the signal value decreases with the increase of the concentration (a dropping section is defined), but the growth rate A continues to increase with the increase of the concentration (as shown in Fig. 2). RLU1 (1 min), RLU2 (5 min), RLU3 (10 min), RLU4 (20 min), and A of the sample to be detected are obtained by detection with the method of the present disclosure. A standard curve of RLU2 (5 min) and a standard curve of A in a full measurement range (for example, 0-3,276,800 ng/ml) are plotted. It is determined whether the concentration is in the rising section or in the dropping section based on the value of A of the analyte, and then an exact concentration is calculated by putting RLU2 (5 min) of the analyte into the corresponding standard curve.

Alternatively, RLU2 (5 min) and RLU1 (1 min) of the sample to be detected obtained by performing two times of value reading are selected, and an RLU growth rate A is calculated based on an equation A=(RLU2/RLU1-1)X 100% as an index for determining a concentration section of the sample. As can be seen from Table 1 and Fig. 1, the signal value increases with the increase of the concentration before the concentration increases to 51,200 ng/ml (a rising section is defined); and then the signal value decreases with the increase of the concentration (a dropping section is defined), but the growth rate A continues to increase with the increase of the concentration (as shown in Fig. 2). RLU1 (1 min) and RLU2 (5 min) of the sample to be detected are detected by the method of the present disclosure, and the growth rate A is calculated. If the growth rate A of the sample is located in the rising section, no value reading is performed, and the concentration is calculated by directly putting the signal value read at 1 min of the sample to be detected into the standard curve between RLU1 (1 min) and the concentration. If the growth rate A of the sample is located in the dropping section, multiple times of value reading are performed, and the concentration is calculated by putting the signal value read of the sample to be detected into the standard curve between RLU4 (20 min) and the concentration.

### Example 2: Detection of a human chorionic gonadotropin and β subunit (HCG+β) sample by a conventional method and by a method of the present disclosure respectively

A human chorionic gonadotropin and β subunit (HCG+β) detection kit (chemiluminescence) produced by Shanghai Beyond Biotech Co., Ltd was used to measure a content of human chorionic gonadotropin and β subunit in a sample.

Gradient dilution was performed on high-concentration antigens of human chorionic gonadotropin and β subunit. Signal values of samples having different concentrations of human chorionic gonadotropin and β subunit were detected by a conventional method and by a method of the present disclosure, respectively.

Detection by the conventional method: An analyte sample with a known concentration was mixed with a reagent 1 (a solution of a acceptor bonded to a mouse monoclonal antibody) and a reagent 2 (a solution of a mouse monoclonal antibody bonded to biotin), and a resulting mixture was incubated at 37°C for 15 min. Then, a reagent 3 (a solution of a donor bonded to streptavidin) was added, and a resulting mixture was incubated at 37°C for 15 min. A photon counter was used to read a signal value which was marked as RLU. Results were shown in Table 2.

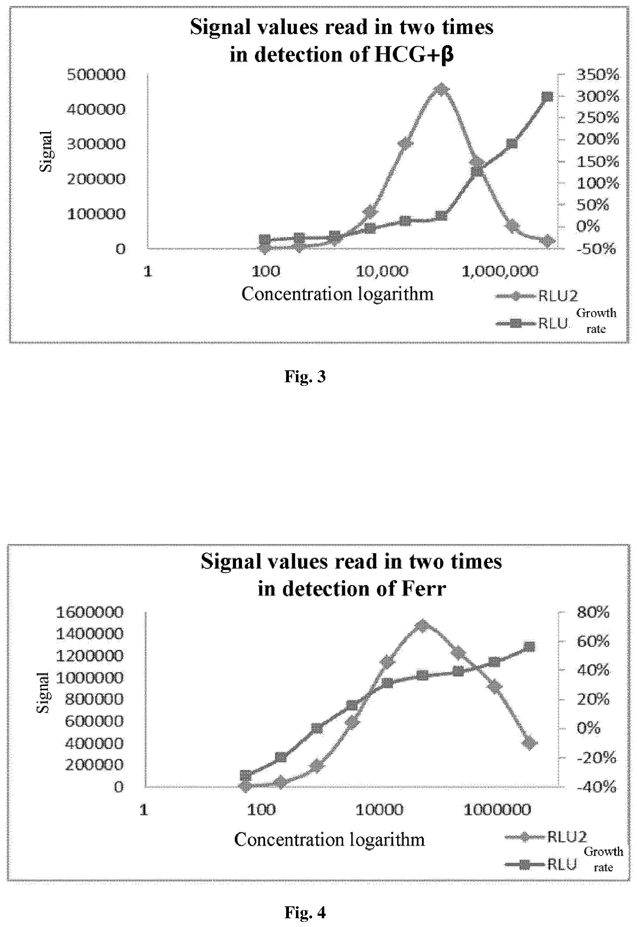

Detection by performing two times of value reading according to the method of the present disclosure: An analyte sample with a known concentration was mixed with a reagent 1 (a light-emitting antibody, namely light-emitting particles coated with a mouse monoclonal antibody) and a reagent 2 (a biotin-labeled antibody, namely a mouse monoclonal antibody labeled with biotin), and a resulting mixture was incubated at 37°C for 15 min. Then, a LiCA general-purpose solution (light-sensitive particles labeled with streptavidin) was added, and a resulting mixture was incubated at 37°C for 3 min. A signal value RLU1 (1 min) was read. After that, the mixture was incubated again at 37°C for 7 min, and a signal value RLU2 was read. A growth rate A from the signal value read at a first time to the signal value read at a second time was calculated based on an equation A=(RLU2/RLU1-1) × 100%. Detection results were shown in Table 2 and Fig. 3.

**Table 2:**

| Sample numbers | Concentrations (mIU/ml) | Conventional detection results | Detection results by method of the present discolsure | | |
|---|---|---|---|---|---|
| | | RLU | RLU1 | RLU2 | Growth rates A |
| 1# | 100 | 4783 | 6073 | 4217 | -31% |
| 2# | 400 | 16153 | 22297 | 15630 | -30% |
| 3# | 1,600 | 68730 | 85405 | 66818 | -22% |
| 4# | 6,400 | 282534 | 283179 | 263684 | -7% |
| 5# | 25,600 | 844830 | 663207 | 741788 | 12% |
| 6# | 102,400 | 1162159 | 855280 | 1044928 | 22% |
| 7# | 409,600 | 395703 | 272396 | 378001 | 39% |
| 8# | 1,638,400 | 97248 | 56037 | 88323 | 58% |
| 9# | 6,553,600 | 23462 | 13174 | 21321 | 62% |

As can be seen from Table 2, when the concentration is in a range from 100 mIU/ml to 102,400 mIU/ml, the signal value increases with the increase of the concentration; and when the concentration continues to increase, the signal value decreases with the increase of the concentration of human chorionic gonadotropin and β subunit. That is, when the concentration is larger than 102,400 mIU/ml (this concentration was defined as an HD-HOOK inflection point, and a growth rate thereof was defined as A₀), the HD-HOOK effect occurs. In a conventional detection, for a sample with an antigen concentration higher than this detection range, a detected concentration will be low (detected concentrations are all less than 102,400 mIU/ml).

The method of the present disclosure broadens a detection range and indicates an HD-HOOK sample or a sample that is beyond the detection range by way of selecting two signal values. Each sample is detected for two times to obtain two signal values RLU1 and RLU2. A growth rate A from the signal value read at a first time to the signal value read at a second time calculated based on an equation A= (RLU2/RLU1-1)×100% is used as an index for determining a concentration section of the sample. As can be seen from Table 2 and Fig. 3, the signal value increases with the increase of the concentration before the concentration increases to 51,200 ng/ml (a rising section is defined); and then the signal value decreases with the increase of the concentration (a dropping section is defined), but the growth rate A continues to increase with the increase of the concentration. RLU1, RLU2, and A of the sample to be detected are obtained by detection with the method of the present disclosure.

A standard curve of RLU2 and a standard curve of A in a full measurement range (for example, 0-6,553,600 mIU/ml) are plotted (as shown in Fig. 3). It is determined whether the concentration is in the rising section or in the dropping section based on the value of A of the analyte, and then an exact concentration is calculated by putting RLU2 of the analyte into the corresponding standard curve.

### Example 3: Detection of a ferritin (Ferr) sample by a conventional method and by a method of the present disclosure respectively

A ferritin (Ferr) detection kit (chemiluminescence) produced by Shanghai Beyond Biotech Co., Ltd was used to measure a content of ferritin in a sample.

Gradient dilution was performed on high-concentration antigens of ferritin. Signal values of samples having different concentrations of ferritin were detected by a conventional method and by a method of the present disclosure, respectively.

Detection by the conventional method: An analyte sample with a known concentration, a reagent 1 (a light-emitting antibody, namely light-emitting particles coated with a mouse monoclonal antibody), and a reagent 2 (a biotin-labeled antibody, namely a mouse monoclonal antibody labeled with biotin) were added into a reaction vessel. and then a resulting mixture was incubated at 37°C for 15 min. After that, a LiCA general-purpose solution (light-sensitive particles labeled with streptavidin) was added, and a resulting mixture was incubated at 37°C for 10 min. A photon counter was used to read a signal value which was marked as RLU. Results were shown in Table 3.

Detection by performing two times of value reading according to the method of the present disclosure: An analyte sample with a known concentration was mixed with a reagent 1 (a light-emitting antibody, namely light-emitting particles coated with a mouse monoclonal antibody) and a reagent 2 (a biotin-labeled antibody, namely a mouse monoclonal antibody labeled with biotin), and a resulting mixture was incubated at 37°C for 15 min. Then, a LiCA general-purpose solution (light-sensitive particles labeled with streptavidin) was added, and a resulting mixture was incubated at 37°C for 3 min. A signal value RLU1 was read. After that, the mixture was incubated again at 37°C for 7 min, and a signal value RLU2 was read. A growth rate A from the signal value read at a first time to the signal value read at a second time was calculated based on an equation A=(RLU2/RLU1-1)× 100%. Detection results were shown in Table 3 and Fig. 4.

**Table 3:**

| Sample numbers | Concentrations (ng/ml) | Conventional detection results | Detection results by method of the present discolsure | | |
|---|---|---|---|---|---|
| | | RLU | RLU1 | RLU2 | Growth rates A |
| 1# | 50 | 29374 | 36982 | 29226 | -21% |
| 2# | 200 | 130237 | 139538 | 118990 | -15% |
| 3# | 800 | 514946 | 470907 | 467257 | -1% |
| 4# | 3200 | 1631905 | 1219373 | 1437994 | 18% |
| 5# | 12800 | 2994937 | 2280569 | 2821545 | 24% |
| 6# | 51200 | 3402607 | 2578903 | 3163238 | 23% |
| 7# | 204800 | 3272694 | 2314036 | 2907751 | 26% |
| 8# | 819200 | 2437951 | 1645331 | 2165755 | 32% |
| 9# | 3276800 | 1002072 | 633495 | 948425 | 50% |

As can be seen from Table 3, when the concentration is in a range from 50 ng/ml to 51,200 ng/ml, the signal value increases with the increase of the concentration; and when the concentration continues to increase, the signal value decreases with the increase of the concentration of ferritin. That is, when the concentration is larger than 51,200 ng/ml (this concentration was defined as an HD-HOOK inflection point, and a growth rate thereof was defined as A₀), the HD-HOOK effect occurs. In a conventional detection, for a sample with an antigen concentration higher than this detection range, a detected concentration will be low (detected concentrations are all less than 51,200 ng/ml).

The method of the present disclosure broadens a detection range and indicates an HD-HOOK sample or a sample that is beyond the detection range by way of selecting two signal values. Each sample is detected for two times to obtain two signal values RLU1 and RLU2. A growth rate A from the signal value read at a first time to the signal value read at a second time calculated based on an equation A= (RLU2/RLU1-1)×100% is used as an index for determining a concentration section of the sample. As can be seen from Table 3 and Fig. 4, the signal value increases with the increase of the concentration before the concentration increases to 51,200 ng/ml (a rising section is defined); and then the signal value decreases with the increase of the concentration (a dropping section is defined), but the growth rate A continues to increase with the increase of the concentration. RLU1, RLU2, and A of the sample to be detected are obtained by detection with the method of the present disclosure.

A standard curve of RLU2 and a standard curve of A in a full measurement range (for example, 0-3,276,800 ng/ml) are plotted (as shown in Fig. 4). It is determined whether the concentration is in the rising section or in the dropping section based on the value of A of the analyte, and then an exact concentration is calculated by putting RLU2 of the analyte into the corresponding standard curve.

### Example 4: Detection of an HIV antibody (anti-HIV) sample by a conventional method and by a method of the present disclosure respectively

An HIV antibody (anti-HIV) detection kit (chemiluminescence) produced by Shanghai Beyond Biotech Co., Ltd was used to measure a content of HIV antibody in a sample.

Gradient dilution was performed on high-concentration antigens of HIV antibody. Signal values of samples having different concentrations of HIV antibody were detected by a conventional method and by a method of the present disclosure, respectively.

Detection by the conventional method: An analyte sample with a known concentration, a reagent 1 (a light-emitting reagent, namely light-emitting particles coated with an HIV antigen), and a reagent 2 (a biotin reagent, namely an HIV antigen labeled with biotin) were added into a reaction vessel. and then a resulting mixture was incubated at 37°C for 15 min. After that, a LiCA general-purpose solution (light-sensitive particles labeled with streptavidin) was added, and a resulting mixture was incubated at 37°C for 10 min. A photon counter was used to read a signal value which was marked as RLU. Results were shown in Table 4.

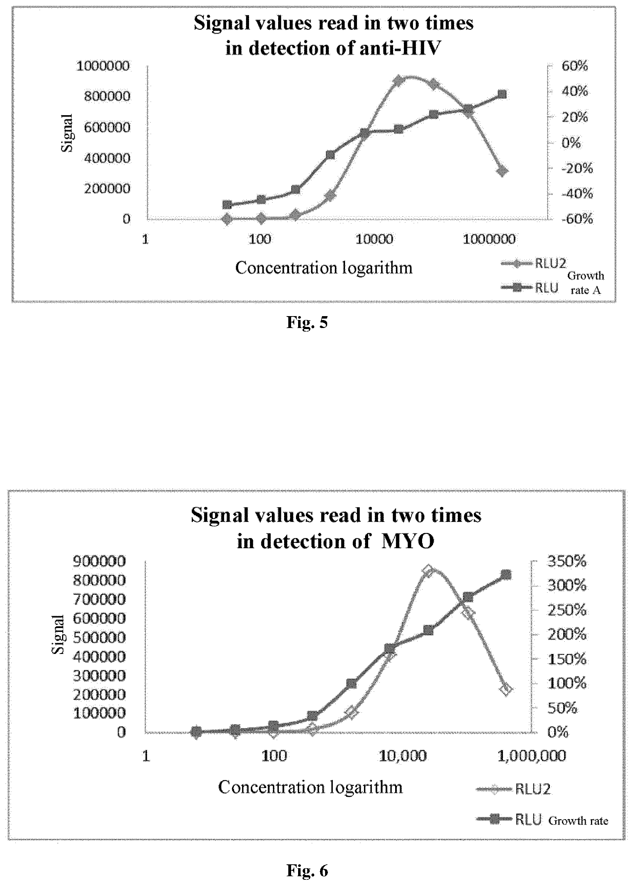

Detection by performing two times of value reading according to the method of the present disclosure: An analyte sample with a known concentration was mixed with a reagent 1 (a light-emitting reagent, namely light-emitting particles coated with an HIV antigen) and a reagent 2 (a biotin reagent, namely an HIV antigen labeled with biotin), and a resulting mixture was incubated at 37°C for 15 min. Then, a LiCA general-purpose solution (light-sensitive particles labeled with streptavidin) was added, and a resulting mixture was incubated at 37°C for 3 min. A signal value RLU1 was read. After that, the mixture was incubated again at 37°C for 7 min, and a signal value RLU2 was read. A growth rate A from the signal value read at a first time to the signal value read at a second time was calculated based on an equation A=(RLU2/RLU1-1) × 100%. Detection results were shown in Table 4 and Fig. 5.

**Table 4:**

| Sample numbers | Concentrations (ng/ml) | Conventional detection results | Detection results by method of the present discolsure | | |
|---|---|---|---|---|---|
| | | RLU | RLU1 | RLU2 | Growth rates A |
| 1# | 25 | 3187 | 6172 | 3086 | -50% |
| 2# | 100 | 13310 | 21969 | 11467 | -48% |
| 3# | 400 | 66789 | 97480 | 61403 | -37% |
| 4# | 1600 | 403890 | 426498 | 380495 | -11% |
| 5# | 6400 | 1591893 | 1301691 | 1488670 | 14% |
| 6# | 25600 | 2683158 | 1921236 | 2331572 | 21% |
| 7# | 102400 | 2497961 | 1829868 | 2258869 | 23% |
| 8# | 409600 | 1854742 | 1305503 | 1712345 | 31% |
| 9# | 1638400 | 820651 | 539365 | 778920 | 44% |

As can be seen from Table 4, when the concentration is in a range from 25 ng/ml to 25,600 ng/ml, the signal value increases with the increase of the concentration; and when the concentration continues to increase, the signal value decreases with the increase of the concentration of HIV antibody. That is, when the concentration is larger than 25,600 ng/ml (this concentration was defined as an HD-HOOK inflection point, and a growth rate thereof was defined as A₀), the HD-HOOK effect occurs. In a conventional detection, for a sample with an antigen concentration higher than this detection range, a detected concentration will be low (detected concentrations are all less than 256,00 ng/ml).

The method of the present disclosure broadens a detection range and indicates an HD-HOOK sample or a sample that is beyond the detection range by way of selecting two signal values. Each sample is detected for two times to obtain two signal values RLU1 and RLU2. A growth rate A from the signal value read at a first time to the signal value read at a second time calculated based on an equation A= (RLU2/RLU1-1)×100% is used as an index for determining a concentration section of the sample. As can be seen from Table 4 and Fig. 5, the signal value increases with the increase of the concentration before the concentration increases to 25,600 ng/ml (a rising section is defined); and then the signal value decreases with the increase of the concentration (a dropping section is defined), but the growth rate A continues to increase with the increase of the concentration. RLU1, RLU2, and A of the sample to be detected are obtained by detection with the method of the present disclosure.

A standard curve of RLU2 and a standard curve of A in a full measurement range (for example, 0-1,638,400ng/ml) are plotted (as shown in Fig. 5). It is determined whether the concentration is in the rising section or in the dropping section based on the value of A of the analyte, and then an exact concentration is calculated by putting RLU2 of the analyte into the corresponding standard curve.

### Example 5: Detection of a myoglobin (MYO) sample by a conventional method and by a method of the present disclosure respectively

A myoglobin (MYO) detection kit (chemiluminescence) produced by Shanghai Beyond Biotech Co., Ltd was used to measure a content of myoglobin in a sample.

Gradient dilution was performed on high-concentration antigens of myoglobin. Signal values of samples having different concentrations of myoglobin were detected by a conventional method and by a method of the present disclosure, respectively.

Detection by the conventional method: An analyte sample with a known concentration, a reagent 1 (a light-emitting antibody, namely light-emitting particles coated with a mouse monoclonal antibody), and a reagent 2 (a biotin-labeled antibody, namely a mouse monoclonal antibody labeled with biotin) were added into a reaction vessel. and then a resulting mixture was incubated at 37°C for 15 min. After that, a LiCA general-purpose solution (light-sensitive particles labeled with streptavidin) was added, and a resulting mixture was incubated at 37°C for 10 min. A photon counter was used to read a signal value which was marked as RLU. Results were shown in Table 5.

Detection by performing two times of value reading according to the method of the present disclosure: An analyte sample with a known concentration was mixed with a reagent 1 (a light-emitting antibody, namely light-emitting particles coated with a mouse monoclonal antibody) and a reagent 2 (a biotin-labeled antibody, namely a mouse monoclonal antibody labeled with biotin), and a resulting mixture was incubated at 37°C for 15 min. Then, a LiCA general-purpose solution (light-sensitive particles labeled with streptavidin) was added, and a resulting mixture was incubated at 37°C for 3 min. A signal value RLU1 was read. After that, the mixture was incubated again at 37°C for 7 min, and a signal value RLU2 was read. A growth rate A from the signal value read at a first time to the signal value read at a second time was calculated based on an equation A=(RLU2/RLU1-1)× 100%. Detection results were shown in Table 5 and Fig. 6.

**Table 5:**

| Sample numbers | Concentrations (ng/ml) | Conventional detection results | Detection results by method of the present discolsure | | |
|---|---|---|---|---|---|
| | | RLU | RLU1 | RLU2 | Growth rates A |
| 1# | 6 | 4087 | 3001 | 3124 | 4% |
| 2# | 25 | 5904 | 4450 | 4679 | 5% |
| 3# | 100 | 13489 | 10081 | 11069 | 10% |
| 4# | 400 | 44629 | 35422 | 46248 | 31% |
| 5# | 1,600 | 167251 | 130712 | 261070 | 100% |
| 6# | 6,400 | 468674 | 374761 | 1025554 | 174% |
| 7# | 25,600 | 832315 | 677602 | 2060119 | 204% |
| 8# | 102,400 | 539604 | 417102 | 1574654 | 278% |
| 9# | 409,600 | 179718 | 134668 | 549601 | 308% |

As can be seen from Table 5, when the concentration is in a range from 6 ng/ml to 25,600 ng/ml, the signal value increases with the increase of the concentration; and when the concentration continues to increase, the signal value decreases with the increase of the concentration of HIV antibody. That is, when the concentration is larger than 25,600 ng/ml (this concentration was defined as an HD-HOOK inflection point, and a growth rate thereof was defined as A₀), the HD-HOOK effect occurs. In a conventional detection, for a sample with an antigen concentration higher than this detection range, a detected concentration will be low (detected concentrations are all less than 25,600 ng/ml).

The method of the present disclosure broadens a detection range and indicates an HD-HOOK sample or a sample that is beyond the detection range by way of selecting two signal values. Each sample is detected for two times to obtain two signal values RLU1 and RLU2. A growth rate A from the signal value read at a first time to the signal value read at a second time calculated based on an equation A= (RLU2/RLU1-1)×100% is used as an index for determining a concentration section of the sample. As can be seen from Table 5 and Fig. 6, the signal value increases with the increase of the concentration before the concentration increases to 25,600 ng/ml (a rising section is defined); and then the signal value decreases with the increase of the concentration (a dropping section is defined), but the growth rate A continues to increase with the increase of the concentration. RLU1, RLU2, and A of the sample to be detected are obtained by detection with the method of the present disclosure.

A standard curve of RLU2 and a standard curve of A in a full measurement range (for example, 0-409,600 ng/ml) are plotted (as shown in Fig. 6). It is determined whether the concentration is in the rising section or in the dropping section based on the value of A of the analyte, and then an exact concentration is calculated by putting RLU2 of the analyte into the corresponding standard curve.

### Example 6: Detection of an N-terminal atrium natriuretic peptide (NT-proBNP) sample by a conventional method and by a method of the present disclosure respectively

An N-terminal atrium natriuretic peptide (NT-proBNP) detection kit (chemiluminescence) produced by Shanghai Beyond Biotech Co., Ltd was used to measure a content of N-terminal atrium natriuretic peptide in a sample.

Gradient dilution was performed on high-concentration antigens of N-terminal atrium natriuretic peptide. Signal values of samples having different concentrations of N-terminal atrium natriuretic peptide were detected by a conventional method and by a method of the present disclosure, respectively.

Detection by the conventional method: An analyte sample with a known concentration, a reagent 1 (a light-emitting antibody, namely light-emitting particles coated with a mouse monoclonal antibody), and a reagent 2 (a biotin-labeled antibody, namely a mouse monoclonal antibody labeled with biotin) were added into a reaction vessel. and then a resulting mixture was incubated at 37°C for 15 min. After that, a LiCA general-purpose solution (light-sensitive particles labeled with streptavidin) was added, and a resulting mixture was incubated at 37°C for 10 min. A photon counter was used to read a signal value which was marked as RLU. Results were shown in Table 6.

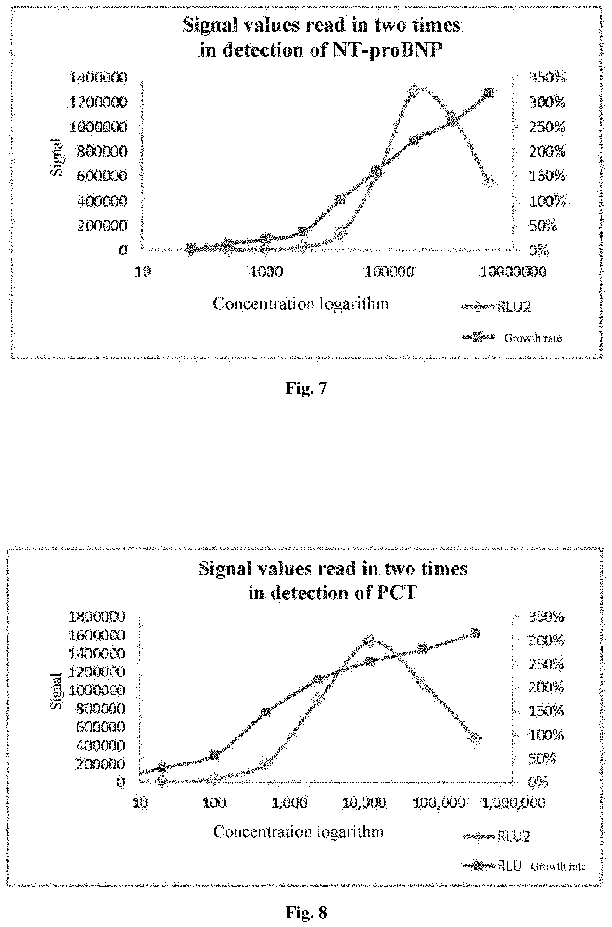

Detection by performing two times of value reading according to the method of the present disclosure: An analyte sample with a known concentration was mixed with a reagent 1 (a light-emitting antibody, namely light-emitting particles coated with a mouse monoclonal antibody) and a reagent 2 (a biotin-labeled antibody, namely a mouse monoclonal antibody labeled with biotin), and a resulting mixture was incubated at 37°C for 15 min. Then, a LiCA general-purpose solution (light-sensitive particles labeled with streptavidin) was added, and a resulting mixture was incubated at 37°C for 3 min. A signal value RLU1 was read. After that, the mixture was incubated again at 37°C for 7 min, and a signal value RLU2 was read. A growth rate A from the signal value read at a first time to the signal value read at a second time was calculated based on an equation A=(RLU2/RLU1-1)× 100%. Detection results were shown in Table 6 and Fig. 7.

**Table 6:**

| Sample numbers | Concentrations (pg/ml) | Conventional detection results | Detection results by method of the present discolsure | | |
|---|---|---|---|---|---|
| | | RLU | RLU1 | RLU2 | Growth rates A |
| 1# | 62.5 | 6022 | 4751 | 4847 | 2% |
| 2# | 250 | 8162 | 6507 | 7053 | 8% |
| 3# | 1000 | 19752 | 16182 | 19168 | 18% |
| 4# | 4000 | 71440 | 50520 | 71309 | 41% |
| 5# | 16000 | 211974 | 163755 | 337723 | 106% |
| 6# | 64000 | 750435 | 587531 | 1555828 | 165% |
| 7# | 256000 | 1327403 | 1011360 | 3203183 | 217% |
| 8# | 1024000 | 923568 | 735261 | 2686846 | 265% |
| 9# | 4096000 | 424535 | 322636 | 1343679 | 316% |

As can be seen from Table 6, when the concentration is in a range from 62.5 pg/ml to 256,000 pg/ml, the signal value increases with the increase of the concentration; and when the concentration continues to increase, the signal value decreases with the increase of the concentration of N-terminal atrium natriuretic peptide. That is, when the concentration is larger than 256,000 pg/ml (this concentration was defined as an HD-HOOK inflection point, and a growth rate thereof was defined as A₀), the HD-HOOK effect occurs. In a conventional detection, for a sample with an antigen concentration higher than this detection range, a detected concentration will be low (detected concentrations are all less than 256,000 pg/ml).

The method of the present disclosure broadens a detection range and indicates an HD-HOOK sample or a sample that is beyond the detection range by way of selecting two signal values. Each sample is detected for two times to obtain two signal values RLU1 and RLU2. A growth rate A from the signal value read at a first time to the signal value read at a second time calculated based on an equation A= (RLU2/RLU1-1)×100% is used as an index for determining a concentration section of the sample. As can be seen from Table 6 and Fig. 7, the signal value increases with the increase of the concentration before the concentration increases to 256,000 pg/ml (a rising section is defined); and then the signal value decreases with the increase of the concentration (a dropping section is defined), but the growth rate A continues to increase with the increase of the concentration. RLU1, RLU2, and A of the sample to be detected are obtained by detection with the method of the present disclosure.

A standard curve of RLU2 and a standard curve of A in a full measurement range (for example, 0-4,096,000 pg/ml) are plotted (as shown in Fig. 7). It is determined whether the concentration is in the rising section or in the dropping section based on the value of A of the analyte, and then an exact concentration is calculated by putting RLU2 of the analyte into the corresponding standard curve.

### Example 7: Detection of a procalcitonin (PCT) sample by a conventional method and by a method of the present disclosure respectively

A procalcitonin (PCT) detection kit (chemiluminescence) produced by Shanghai Beyond Biotech Co., Ltd was used to measure a content of procalcitonin in a sample.

Gradient dilution was performed on high-concentration antigens of procalcitonin. Signal values of samples having different concentrations of procalcitonin were detected by a conventional method and by a method of the present disclosure, respectively.

Detection by the conventional method: An analyte sample with a known concentration, a reagent 1 (a light-emitting antibody, namely light-emitting particles coated with a mouse monoclonal antibody), and a reagent 2 (a biotin-labeled antibody, namely a mouse monoclonal antibody labeled with biotin) were added into a reaction vessel. and then a resulting mixture was incubated at 37°C for 15 min. After that, a LiCA general-purpose solution (light-sensitive particles labeled with streptavidin) was added, and a resulting mixture was incubated at 37°C for 10 min. A photon counter was used to read a signal value which was marked as RLU. Results were shown in Table 7.

Detection by performing two times of value reading according to the method of the present disclosure: An analyte sample with a known concentration was mixed with a reagent 1 (a light-emitting antibody, namely light-emitting particles coated with a mouse monoclonal antibody) and a reagent 2 (a biotin-labeled antibody, namely a mouse monoclonal antibody labeled with biotin), and a resulting mixture was incubated at 37°C for 15 min. Then, a LiCA general-purpose solution (light-sensitive particles labeled with streptavidin) was added, and a resulting mixture was incubated at 37°C for 3 min. A signal value RLU1 was read. After that, the mixture was incubated again at 37°C for 7 min, and a signal value RLU2 was read. A growth rate A from the signal value read at a first time to the signal value read at a second time was calculated based on an equation A=(RLU2/RLU1-1)× 100%. Detection results were shown in Table 7 and Fig. 8.

**Table 7:**

| Sample numbers | Concentrations (ng/ml) | Conventional detection results | Detection results by method of the present discolsure | | |
|---|---|---|---|---|---|
| | | RLU | RLU1 | RLU2 | Growth rates A |
| 1# | 1 | 6494 | 5677 | 6306 | 11% |
| 2# | 4 | 9300 | 8700 | 9265 | 6% |
| 3# | 20 | 23184 | 19058 | 25090 | 32% |
| 4# | 100 | 70813 | 65143 | 103267 | 59% |
| 5# | 500 | 252498 | 218813 | 537843 | 146% |
| 6# | 2,500 | 785933 | 716506 | 2206900 | 208% |
| 7# | 12,500 | 1220448 | 1104767 | 3872725 | 251% |
| 8# | 62,500 | 814576 | 708063 | 2664094 | 276% |
| 9# | 312,500 | 364549 | 293546 | 1198940 | 308% |

As can be seen from Table 7, when the concentration is in a range from 1 ng/ml to 12,500 ng/ml, the signal value increases with the increase of the concentration; and when the concentration continues to increase, the signal value decreases with the increase of the concentration of procalcitonin. That is, when the concentration is larger than 12,500 ng/ml (this concentration was defined as an HD-HOOK inflection point, and a growth rate thereof was defined as A₀), the HD-HOOK effect occurs. In a conventional detection, for a sample with an antigen concentration higher than this detection range, a detected concentration will be low (detected concentrations are all less than 12,500 ng/ml).

The method of the present disclosure broadens a detection range and indicates an HD-HOOK sample or a sample that is beyond the detection range by way of selecting two signal values. Each sample is detected for two times to obtain two signal values RLU1 and RLU2. A growth rate A from the signal value read at a first time to the signal value read at a second time calculated based on an equation A= (RLU2/RLU1-1)×100% is used as an index for determining a concentration section of the sample. As can be seen from Table 7 and Fig. 8, the signal value increases with the increase of the concentration before the concentration increases to 12,500 ng/ml (a rising section is defined); and then the signal value decreases with the increase of the concentration (a dropping section is defined), but the growth rate A continues to increase with the increase of the concentration. RLU1, RLU2, and A of the sample to be detected are obtained by detection with the method of the present disclosure.

A standard curve of RLU2 and a standard curve of A in a full measurement range (for example, 0-312,500 ng/ml) are plotted (as shown in Fig. 8). It is determined whether the concentration is in the rising section or in the dropping section based on the value of A of the analyte, and then an exact concentration is calculated by putting RLU2 of the analyte into the corresponding standard curve.

### Example 8: Detection of a troponin I (cTnI) sample by a conventional method and by a method of the present disclosure respectively

A troponin I (cTnI) detection kit (chemiluminescence) produced by Shanghai Beyond Biotech Co., Ltd was used to measure a content of troponin I in a sample.

Gradient dilution was performed on high-concentration antigens of troponin I. Signal values of samples having different concentrations of troponin I were detected by a conventional method and by a method of the present disclosure, respectively.

Detection by the conventional method: An analyte sample with a known concentration, a reagent 1 (a light-emitting antibody, namely light-emitting particles coated with a mouse monoclonal antibody), and a reagent 2 (a biotin-labeled antibody, namely a mouse monoclonal antibody labeled with biotin) were added into a reaction vessel. and then a resulting mixture was incubated at 37°C for 15 min. After that, a LiCA general-purpose solution (light-sensitive particles labeled with streptavidin) was added, and a resulting mixture was incubated at 37°C for 10 min. A photon counter was used to read a signal value which was marked as RLU. Results were shown in Table 8.

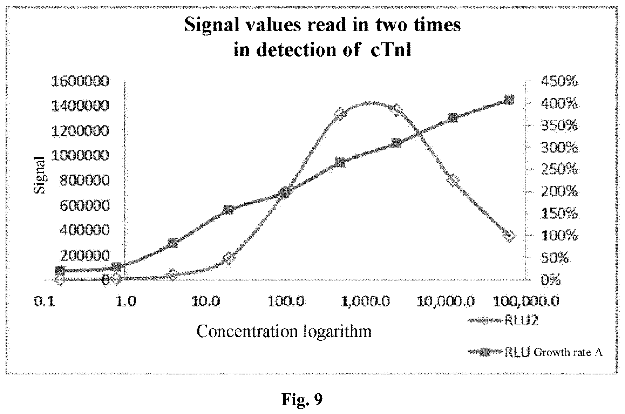

Detection by performing two times of value reading according to the method of the present disclosure: An analyte sample with a known concentration was mixed with a reagent 1 (a light-emitting antibody, namely light-emitting particles coated with a mouse monoclonal antibody) and a reagent 2 (a biotin-labeled antibody, namely a mouse monoclonal antibody labeled with biotin), and a resulting mixture was incubated at 37°C for 15 min. Then, a LiCA general-purpose solution (light-sensitive particles labeled with streptavidin) was added, and a resulting mixture was incubated at 37°C for 3 min. A signal value RLU1 was read. After that, the mixture was incubated again at 37°C for 7 min, and a signal value RLU2 was read. A growth rate A from the signal value read at a first time to the signal value read at a second time was calculated based on an equation A=(RLU2/RLU1-1)× 100%. Detection results were shown in Table 8 and Fig. 9.

**Table 8:**

| Sample numbers | Concentrations (ng/ml) | Conventional detection results | Detection results by method of the present discolsure | | |
|---|---|---|---|---|---|
| | | RLU | RLU1 | RLU2 | Growth rates A |
| 1# | 0.2 | 7474 | 7047 | 8345 | 18% |
| 2# | 1 | 17361 | 15219 | 19797 | 30% |
| 3# | 4 | 55091 | 52344 | 95548 | 83% |
| 4# | 20 | 190395 | 174092 | 439092 | 152% |
| 5# | 100 | 635772 | 593405 | 1727512 | 191% |
| 6# | 500 | 961652 | 933015 | 3338403 | 258% |
| 7# | 2,500 | 865486 | 839713 | 3200562 | 281% |
| 8# | 12,500 | 455932 | 417650 | 1991517 | 377% |
| 9# | 62,500 | 101783 | 104668 | 549601 | 425% |

As can be seen from Table 8, when the concentration is in a range from 0.2 ng/ml to 500 ng/ml, the signal value increases with the increase of the concentration; and when the concentration continues to increase, the signal value decreases with the increase of the concentration of troponin I. That is, when the concentration is larger than 500 ng/ml (this concentration was defined as an HD-HOOK inflection point, and a growth rate thereof was defined as A₀), the HD-HOOK effect occurs. In a conventional detection, for a sample with an antigen concentration higher than this detection range, a detected concentration will be low (detected concentrations are all less than 500 ng/ml).

The method of the present disclosure broadens a detection range and indicates an HD-HOOK sample or a sample that is beyond the detection range by way of selecting two signal values. Each sample is detected for two times to obtain two signal values RLU1 and RLU2. A growth rate A from the signal value read at a first time to the signal value read at a second time calculated based on an equation A= (RLU2/RLU1-1)×100% is used as an index for determining a concentration section of the sample. As can be seen from Table 8 and Fig. 9, the signal value increases with the increase of the concentration before the concentration increases to 500 ng/ml (a rising section is defined); and then the signal value decreases with the increase of the concentration (a dropping section is defined), but the growth rate A continues to increase with the increase of the concentration. RLU1, RLU2, and A of the sample to be detected are obtained by detection with the method of the present disclosure.

A standard curve of RLU2 and a standard curve of A in a full measurement range (for example, 0-62,500 ng/ml) are plotted (as shown in Fig. 9). It is determined whether the concentration is in the rising section or in the dropping section based on the value of A of the analyte, and then an exact concentration is calculated by putting RLU2 of the analyte into the corresponding standard curve.

## Claims

1. A chemiluminescence immune analytical method, comprising the following steps of:
(1) mixing a sample to be detected suspected of containing a target molecule to be detected with a reagent required for a chemiluminescence immune reaction to react so as to form a mixture to be detected;
(2) initiating the mixture to be detected for t times successively to generate chemiluminescence, and recording a signal value of the chemiluminescence for n times, wherein a signal value of the chemiluminescence recorded at the n^{th} time is marked as RLUn;
(3) selecting any two signal values from n-time recorded signal values of the chemiluminescence, marking the two signal values respectively as RLUm and RLUk, and marking a growth rate from RLUm to RLUk as A;
(4) plotting a standard curve based on a growth rate A' from RLUm' to RLUk' in any two reactions at step (2) and step (3) with respect to a series of standard substances with known concentrations containing the target molecule to be detected; and
(5) comparing the growth rate A obtained at step (3) with the standard curve obtained at step (4) to determine a concentration of the target molecule to be detected,
wherein t, n, m, and k are all natural numbers larger than 0, and k<m≤n≤t, n≥2.

2. The method according to claim 1, wherein the growth rate A=(RLUm/RLUk-1)×100%;and/or, wherein n is larger than 2.

3. The method according to claim 1, wherein step (5) is: comparing the growth rate A obtained at step (3) with the standard curve obtained at step (4), wherein
when the growth rate A is located in a rising section of the standard curve, step (2) is stopped, and a concentration of the target molecule to be detected is calculated by directly putting RLUp read at the P^{th} time into the standard curve; and
when the growth rate A is located in a dropping section of the standard curve, the step (2) is continued, and a concentration of the target molecule to be detected is calculated by putting RLUq of the reaction read at the q^{th} time into the standard curve,
wherein p and q are both natural numbers larger than 0, and p≤q≤n;
preferably, wherein p is 1, and q is n.

4. The method according to claim 1, wherein at step (1), the reagent required for the chemiluminescence reaction comprises a acceptor reagent and a donor reagent, wherein
the donor reagent comprises a donor, and the donor is capable of generating singlet oxygen in an initiated state; and
the acceptor reagent comprises a acceptor, and the acceptor is capable of reacting with the singlet oxygen so as to generate a detectable chemiluminescence signal value.

5. The method according to claim 1, wherein the method specifically comprises the following steps of:
(a1) mixing a sample to be detected suspected of containing an antigen (or an antibody) to be detected with a acceptor reagent and performing a first incubation, and mixing a mixed liquid obtained from the first incubation with a donor reagent and performing a second incubation so as to form a mixture to be detected;
(a2) initiating the mixture to be detected for t times successively with irradiation of a red laser beam of 600-700 nm to generate chemiluminescence, and recording a signal value of the chemiluminescence for n times with a detection wavelength of 520-620 nm, wherein a signal value of the chemiluminescence recorded at the n^{th} time is marked as RLUn;
(a3) selecting any two signal values from n-time recorded signal values of the chemiluminescence, marking the two signal values respectively as RLUm and RLUk, and marking a growth rate from RLUm to RLUk as A, the growth rate A=(RLUm/RLUk-1)×100%;
(a4) plotting a standard curve based on a growth rate A' from RLUm' to RLUk' in any two reactions at step (a2) and step (a3) with respect to a series of positive control samples with known concentrations containing the target molecule to be detected; and
(a5) comparing the growth rate A obtained at step (a3) with the standard curve obtained at step (a4) to determine a concentration of the target molecule to be detected,
wherein t, n, m, and k are all natural numbers larger than 0, and k<m≤n≤t, n≥2.

6. The method according to claim 5, wherein step (a5) is: determining, based a value of A, whether the concentration of the target molecule to be detected is located in a rising section or a dropping section of the standard curve, and then calculating the concentration of the target molecule to be detected by putting RLUm of the target molecule to be detected into a corresponding standard curve.

7. The method according to claim 5, wherein step (a5) is: comparing the growth rate A with the standard curve, wherein
when the growth rate A is located in a rising section of the standard curve, step (a2) is stopped, and a concentration of the target molecule to be detected is calculated by directly putting RLUp read at the P^{th} time into the standard curve; and
when the growth rate A is located in a dropping section of the standard curve, the step (a2) is continued, and a concentration of the target molecule to be detected is calculated by putting RLUq of the reaction read at the q^{th} time into the standard curve,
wherein p and q are both natural numbers larger than 0, and p≤q≤n.

8. A system using the chemiluminescence immune analytical method, comprising:
a reaction device, which is configured to conduct a chemiluminescence reaction therein;
an initiating and recording device, which is configured to initiate a mixture to be detected for t times successively to generate chemiluminescence, and record a signal value of the chemiluminescence for n times, wherein a signal value of the chemiluminescence recorded at the n^{th} time is marked as RLUn; and which is configured to select any two signal values from n-time recorded signal values of the chemiluminescence, mark the two signal values respectively as RLUm and RLUk, and mark a growth rate from RLUm to RLUk as A; and
a processor, which is configured to plot a standard curve based on a growth rate A' from RLUm' to RLUk' in any two reactions with respect to a series of standard substances with known concentrations containing the target molecule to be detected; and which is configured to compare the growth rate A with the standard curve to determine a concentration of the target molecule to be detected,
wherein t, n, m, and k are all natural numbers larger than 0, and k<m≤n≤t, n≥2.

9. The system according to claim 8, wherein a method of using the system comprises the following steps of:
(1) mixing a sample to be detected suspected of containing a target molecule to be detected with a reagent required for a chemiluminescence reaction to react so as to form a mixture to be detected;
(2) initiating the mixture to be detected for t times successively to generate chemiluminescence, and recording a signal value of the chemiluminescence for n times, wherein a signal value of the chemiluminescence recorded at the n^{th} time is marked as RLUn;
(3) selecting any two signal values from n-time recorded signal values of the chemiluminescence, marking the two signal values respectively as RLUm and RLUk, and marking a growth rate from RLUm to RLUk as A;
(4) plotting a standard curve based on a growth rate A' from RLUm' to RLUk' in any two reactions at step (2) and step (3) with respect to a series of standard substances with known concentrations containing the target molecule to be detected; and
(5) comparing the growth rate A obtained at step (3) with the standard curve obtained at step (4) to determine a concentration of the target molecule to be detected,
wherein t, n, m, and k are all natural numbers larger than 0, and k<m≤n≤t, n≥2.

10. The system according to claim 8, wherein the growth rate A=(RLUm/RLUk-1)×100%; and/or, wherein n is larger than 2.

11. The system according to claim 9, wherein step (5) is: comparing the growth rate A obtained at step (3) with the standard curve obtained at step (4), wherein
when the growth rate A is located in a rising section of the standard curve, step (2) is stopped, and a concentration of the target molecule to be detected is calculated by directly putting RLUp read at the P^{th} time into the standard curve; and
when the growth rate A is located in a dropping section of the standard curve, the step (2) is continued, and a concentration of the target molecule to be detected is calculated by putting RLUq of the reaction read at the q^{th} time into the standard curve,
wherein t, n, m, k, p, and q are all natural numbers larger than 0, and k<m≤n≤t, p≤q≤n, n≥2;
preferably, wherein p is 1, and q is n.

12. Use of a kit comprising a reagent required for a chemiluminescence analysis in a method comprising the following steps:
(1) mixing a sample to be detected suspected of containing a target molecule to be detected with a reagent required for a chemiluminescence reaction to react so as to form a mixture to be detected;
(2) initiating the mixture to be detected for t times successively to generate chemiluminescence, and recording a signal value of the chemiluminescence for n times, wherein a signal value of the chemiluminescence recorded at the n^{th} time is marked as RLUn;
(3) selecting any two signal values from n-time recorded signal values of the chemiluminescence, marking the two signal values respectively as RLUm and RLUk, and marking a growth rate from RLUm to RLUk as A;
(4) plotting a standard curve based on a growth rate A' from RLUm' to RLUk' in any two reactions at step (2) and step (3) with respect to a series of standard substances with known concentrations containing the target molecule to be detected; and
(5) comparing the growth rate A obtained at step (3) with the standard curve obtained at step (4) to determine a concentration of the target molecule to be detected,
wherein t, n, m, and k are all natural numbers larger than 0, and k<m≤n≤t, n≥2.

13. The use of the kit according to claim 12, wherein the method of using the kit comprises the following steps of:
(a1) mixing a sample to be detected suspected of containing an antigen (or an antibody) to be detected with a acceptor reagent and performing a first incubation, and mixing a mixed liquid obtained from the first incubation with a donor reagent and performing a second incubation so as to form a mixture to be detected;
(a2) initiating the mixture to be detected for t times successively with irradiation of a red laser beam of 600-700 nm to generate chemiluminescence, and recording a signal value of the chemiluminescence for n times with a detection wavelength of 520-620 nm, wherein a signal value of the chemiluminescence recorded at the n^{th} time is marked as RLUn;
(a3) selecting any two signal values from n-time recorded signal values of the chemiluminescence, marking the two signal values respectively as RLUm and RLUk, and marking a growth rate from RLUm to RLUk as A, the growth rate A=(RLUm/RLUk-1)×100%;
(a4) plotting a standard curve based on a growth rate A' from RLUm' to RLUk' in any two reactions at step (a2) and step (a3) with respect to a series of positive control samples with known concentrations containing the target molecule to be detected; and
(a5) comparing the growth rate A obtained at step (a3) with the standard curve obtained at step (a4) to determine a concentration of the target molecule to be detected,
wherein t, n, m, and k are all natural numbers larger than 0, and k<m≤n≤t, n≥2.

14. The use of the kit according to claim 13, wherein step (a5) is: determining, based a value of A, whether the concentration of the target molecule to be detected is located in a rising section or a dropping section of the standard curve, and then calculating the concentration of the target molecule to be detected by putting RLUm of the target molecule to be detected into a corresponding standard curve.

15. The use of the kit according to claim 14, wherein step (a5) is: comparing the growth rate A with the standard curve, wherein
when the growth rate A is located in a rising section of the standard curve, step (a2) is stopped, and a concentration of the target molecule to be detected is calculated by directly putting RLUp read at the P^{th} time into the standard curve; and
when the growth rate A is located in a dropping section of the standard curve, the step (a2) is continued, and a concentration of the target molecule to be detected is calculated by putting RLUq of the reaction read at the q^{th} time into the standard curve,
wherein p and q are both natural numbers larger than 0, and p≤q≤n.

## Patentansprüche

1. Immunoanalytisches Chemilumineszenzverfahren, umfassend die folgenden Schritte:
(1) Mischen einer zu detektierenden Probe, die mutmaßlich ein zu detektierendes Zielmolekül enthält, mit einem Reagenz, das für eine Chemilumineszenz-Immunreaktion erforderlich ist, um zu reagieren, so dass eine zu detektierende Mischung gebildet wird;
(2) t-maliges aufeinander folgendes Initiieren der zu detektierenden Mischung, um Chemilumineszenz zu erzeugen, und n-maliges Aufzeichnen eines Signalwerts der Chemilumineszenz, wobei ein Signalwert der zur n. Zeit aufgezeichneten Chemilumineszenz mit RLUn gekennzeichnet wird;
(3) Auswählen von beliebigen zwei Signalwerten von n-malig aufgezeichneten Signalwerten der Chemilumineszenz, wobei die zwei Signalwerte als RLUm beziehungsweise RLUk gekennzeichnet werden, und Kennzeichnen einer Wachstumsrate von RLUm bis RLUk als A;
(4) Plotten einer Standardkurve basierend auf einer Wachstumsrate A' von RLUm' bis RLUk' in beliebigen zwei Reaktionen in Schritt (2) und Schritt (3) in Bezug auf eine Reihe von Standardsubstanzen mit bekannten Konzentrationen, die das zu detektierende Zielmolekül enthalten; und
(5) Vergleichen der in Schritt (3) erhaltenen Wachstumsrate A mit der in Schritt (4) erhaltenen Standardkurve, um eine Konzentration des zu detektierenden Zielmoleküls zu bestimmen,
wobei t, n, m und k alle natürliche Zahlen größer als 0 sind, und k<m≤n≤t, n≥2.

2. Verfahren nach Anspruch 1, wobei die Wachstumsrate A=(RLUm/RLUk-1)x100% ist; und/oder wobei n größer als 2 ist.

3. Verfahren nach Anspruch 1, wobei Schritt (5) ist: Vergleichen der in Schritt (3) erhaltenen Wachstumsrate A mit der in Schritt (4) erhaltenen Standardkurve, wobei
wenn sich die Wachstumsrate A in einem steigenden Abschnitt der Standardkurve befindet, Schritt (2) gestoppt wird, und eine Konzentration des zu detektierenden Zielmoleküls durch direktes Setzen von RLUp, abgelesen zur p. Zeit, in die Standardkurve berechnet wird; und
wenn die Wachstumskurve A sich in einem sinkenden Abschnitt der Standardkurve befindet, der Schritt (2) fortgesetzt wird, und eine Konzentration des zu detektierenden Zielmoleküls durch Setzen von RLUq der Reaktion, abgelesen zur q. Zeit, in die Standardkurve berechnet wird,
wobei p und q beide natürliche Zahlen größer als 0 sind, und p≤q≤n;
wobei vorzugsweise p 1 ist und q n ist.

4. Verfahren nach Anspruch 1, wobei in Schritt (1) das für die Chemilumineszenzreaktion erforderliche Reagenz ein Akzeptorreagenz und ein Donorreagenz umfasst, wobei
das Donorreagenz einen Donor umfasst, und der Donor in der Lage ist, im initiierten Zustand Singulett-Sauerstoff zu erzeugen; und
das Akzeptorreagenz einen Akzeptor umfasst, und der Akzeptor in der Lage ist, mit dem Singulett-Sauerstoff zu reagieren, um so einen detektierbaren Chemilumineszenzsignalwert zu erzeugen.

5. Verfahren nach Anspruch 1, wobei das Verfahren spezifisch die folgenden Schritte umfasst:
(a1) Mischen einer zu detektierenden Probe, die mutmaßlich ein zu detektierendes Antigen (oder einen zu detektierenden Antikörper) enthält, mit einem Akzeptorreagenz, und Durchführen einer ersten Inkubation, und Mischen einer gemischten Flüssigkeit, die aus der ersten Inkubation erhalten wird, mit einem Donorreagenz, und Durchführen einer zweiten Inkubation, um so eine zu detektierende Mischung zu bilden;
(a2) t-maliges aufeinander folgendes Initiieren der zu detektierenden Mischung mit Bestrahlung eines roten Laserstrahls mit 600 bis 700 nm, um Chemilumineszenz zu erzeugen, und n-maliges Aufzeichnen eines Signalwerts der Chemilumineszenz mit einer Detektierungswellenlänge von 520 bis 620 nm, wobei ein Signalwert der zur n. Zeit aufgezeichneten Chemilumineszenz als RLUn gekennzeichnet wird;
(a3) Auswählen von beliebigen zwei Signalwerten von n-malig aufgezeichneten Signalwerten der Chemilumineszenz, wobei die zwei Signalwerte als RLUm beziehungsweise RLUk gekennzeichnet werden, und Kennzeichnen einer Wachstumsrate von RLUm bis RLUk als A, wobei die Wachstumsrate A=(RLUm/RLUk-1)×100 % ist;
(a4) Plotten einer Standardkurve basierend auf einer Wachstumsrate A' von RLUm' bis RLUk' in beliebigen zwei Reaktionen in Schritt (a2) und Schritt (a3) in Bezug auf eine Reihe von Positivkontrollproben mit bekannten Konzentrationen, die das zu detektierende Zielmolekül enthalten; und
(a5) Vergleichen der in Schritt (a3) erhaltenen Wachstumsrate A mit der in Schritt (a4) erhaltenen Standardkurve, um eine Konzentration des zu detektierenden Zielmoleküls zu bestimmen,
wobei t, n, m und k alle natürliche Zahlen größer als 0 sind, und k<m≤n≤t, n≥2.

6. Verfahren nach Anspruch 5, wobei Schritt (a5) ist: Bestimmen basierend auf einem Wert von A, ob die Konzentration des zu detektierenden Zielmoleküls sich in einem steigenden Abschnitt oder einem sinkenden Abschnitt der Standardkurve befindet, und dann Berechnen der Konzentration des zu detektierenden Zielmoleküls, indem RLUm des zu detektierenden Zielmoleküls in eine entsprechende Standardkurve gesetzt wird.

7. Verfahren nach Anspruch 5, wobei Schritt (a5) ist: Vergleichen der Wachstumsrate A mit der Standardkurve, wobei
wenn sich die Wachstumsrate A in einem steigenden Abschnitt der Standardkurve befindet, Schritt (a2) gestoppt wird, und eine Konzentration des zu detektierenden Zielmoleküls durch direktes Setzen von RLUp, abgelesen zur p. Zeit, in die Standardkurve berechnet wird; und
wenn sich die Wachstumsrate A in einem sinkenden Abschnitt der Standardkurve befindet, der Schritt (a2) fortgesetzt wird, und eine Konzentration des zu detektierenden Zielmoleküls durch Setzen von RLUq der Reaktion, abgelesen zur q. Zeit, in die Standardkurve berechnet wird,
wobei p und q beide natürliche Zahlen größer als 0 sind, und p≤q≤n.

8. System, welches das immunanalytische Chemilumineszenzverfahren verwendet, umfassend:
eine Reaktionsvorrichtung, die ausgestaltet ist, um darin eine Chemilumineszenzreaktion durchzuführen;
eine Initiierungs- und Aufzeichnungsvorrichtung, die ausgestaltet ist, um t Mal aufeinander folgend eine zu detektierende Mischung zu initiieren, um Chemilumineszenz zu erzeugen, und n Mal einen Signalwert der Chemilumineszenz aufzuzeichnen, wobei ein Signalwert der zur n. Zeit aufgezeichneten Chemilumineszenz mit RLUn gekennzeichnet wird; und die ausgestaltet ist, um beliebige zwei Signalwerte von n Mal aufgezeichneten Signalwerten der Chemilumineszenz auszuwählen, die zwei Signalwerte als RLUm beziehungsweise RLUk zu kennzeichnen, und eine Wachstumsrate von RLUm bis RLUk mit A zu kennzeichnen; und
einen Prozessor, der ausgestaltet ist, um eine Standardkurve basierend auf einer Wachstumsrate A' von RLUm' bis RLUk' in beliebigen zwei Reaktionen in Bezug auf eine Reihe von Standardsubstanzen mit bekannten Konzentrationen, die das zu detektierende Zielmolekül enthalten, zu plotten; und der ausgestaltet ist, um die Wachstumsrate A mit der Standardkurve zu vergleichen, um eine Konzentration des zu detektierenden Zielmoleküls zu bestimmen,
wobei t, n, m und k alle natürliche Zahlen größer als 0 sind, und k<m≤n≤t, n≥2.

9. System nach Anspruch 8, wobei ein Verfahren zur Verwendung des Systems die folgenden Schritte umfasst:
(1) Mischen einer zu detektierenden Probe, die mutmaßlich ein zu detektierendes Zielmolekül enthält, mit einem Reagenz, das für eine Chemilumineszenzreaktion erforderlich ist, um zu reagieren, so dass eine zu detektierende Mischung gebildet wird;
(2) t-maliges aufeinander folgendes Initiieren der zu detektierenden Mischung, um Chemilumineszenz zu erzeugen, und n-maliges Aufzeichnen eines Signalwerts der Chemilumineszenz, wobei ein Signalwert der zur n. Zeit aufgezeichneten Chemilumineszenz mit RLUn gekennzeichnet wird;
(3) Auswählen von beliebigen zwei Signalwerten von n-malig aufgezeichneten Signalwerten der Chemilumineszenz, wobei die zwei Signalwerte als RLUm beziehungsweise RLUk gekennzeichnet werden, und Kennzeichnen einer Wachstumsrate von RLUm bis RLUk als A;
(4) Plotten einer Standardkurve basierend auf einer Wachstumsrate A' von RLUm' bis RLUk' in beliebigen zwei Reaktionen in Schritt (2) und Schritt (3) in Bezug auf eine Reihe von Standardsubstanzen mit bekannten Konzentrationen, die das zu detektierende Zielmolekül enthalten; und
(5) Vergleichen der in Schritt (3) erhaltenen Wachstumsrate A mit der in Schritt (4) erhaltenen Standardkurve, um eine Konzentration des zu detektierenden Zielmoleküls zu bestimmen,
wobei t, n, m und k alle natürliche Zahlen größer als 0 sind, und k<m≤n≤t, n≥2.

10. System nach Anspruch 8, wobei die Wachstumsrate A=(RLUm/RLUk-1)x100 % ist; und/oder wobei n größer als 2 ist.

11. System nach Anspruch 9, wobei Schritt (5) ist: Vergleichen der in Schritt (3) erhaltenen Wachstumsrate A mit der in Schritt (4) erhaltenen Standardkurve, wobei
wenn sich die Wachstumsrate A in einem steigenden Abschnitt der Standardkurve befindet, Schritt (2) gestoppt wird, und eine Konzentration des zu detektierenden Zielmoleküls durch direktes Setzen von RLUp, abgelesen zur p. Zeit, in die Standardkurve berechnet wird; und
wenn sich die Wachstumsrate A in einem sinkenden Abschnitt der Standardkurve befindet, der Schritt (2) fortgesetzt wird, und eine Konzentration des zu detektierenden Zielmoleküls durch Setzen von RLUq der Reaktion, abgelesen zur q. Zeit, in die Standardkurve berechnet wird,
wobei t, n, m, k, p und q alle natürliche Zahlen größer als 0 sind, und k<m≤n≤t, p≤q≤n, n≥2;
wobei vorzugsweise p 1 ist und q n ist.

12. Verwendung eines Kits, das ein Reagenz umfasst, das für eine Chemilumineszenzanalyse erforderlich ist, in einem Verfahren, umfassend die folgenden Schritte:
(1) Mischen einer zu detektierenden Probe, die mutmaßlich ein zu detektierendes Zielmolekül enthält, mit einem Reagenz, das für eine Chemilumineszenzreaktion erforderlich ist, um zu reagieren, so dass eine zu detektierende Mischung gebildet wird;
(2) t-maliges aufeinander folgendes Initiieren der zu detektierenden Mischung, um Chemilumineszenz zu erzeugen, und n-maliges Aufzeichnen eines Signalwerts der Chemilumineszenz, wobei ein Signalwert der zur n. Zeit aufgezeichneten Chemilumineszenz mit RLUn gekennzeichnet wird;
(3) Auswählen von beliebigen zwei Signalwerten von n-malig aufgezeichneten Signalwerten der Chemilumineszenz, wobei die zwei Signalwerte als RLUm beziehungsweise RLUk gekennzeichnet werden, und Kennzeichnen einer Wachstumsrate von RLUm bis RLUk als A;
(4) Plotten einer Standardkurve basierend auf einer Wachstumsrate A' von RLUm' bis RLUk' in beliebigen zwei Reaktionen in Schritt (2) und Schritt (3) in Bezug auf eine Reihe von Standardsubstanzen mit bekannten Konzentrationen, die das zu detektierende Zielmolekül enthalten; und
(5) Vergleichen der in Schritt (3) erhaltenen Wachstumsrate A mit der in Schritt (4) erhaltenen Standardkurve, um eine Konzentration des zu detektierenden Zielmoleküls zu bestimmen,
wobei t, n, m und k alle natürliche Zahlen größer als 0 sind, und k<m≤n≤t, n≥2.

13. Verwendung des Kits nach Anspruch 12, wobei das Verfahren zur Verwendung des Kits die folgenden Schritte umfasst:
(a1) Mischen einer zu detektierenden Probe, die mutmaßlich ein zu detektierendes Antigen (oder einen zu detektierenden Antikörper) enthält, mit einem Akzeptorreagenz, und Durchführen einer ersten Inkubation, und Mischen einer gemischten Flüssigkeit, die aus der ersten Inkubation erhalten wird, mit einem Donorreagenz, und Durchführen einer zweiten Inkubation, um so eine zu detektierende Mischung zu bilden;
(a2) t-maliges aufeinander folgendes Initiieren der zu detektierenden Mischung mit Bestrahlung eines roten Laserstrahls mit 600 bis 700 nm, um Chemilumineszenz zu erzeugen, und n-maliges Aufzeichnen eines Signalwerts der Chemilumineszenz mit einer Detektierungswellenlänge von 520 bis 620 nm, wobei ein Signalwert der zur n. Zeit aufgezeichneten Chemilumineszenz als RLUn gekennzeichnet wird;
(a3) Auswählen von beliebigen zwei Signalwerten von n-malig aufgezeichneten Signalwerten der Chemilumineszenz, wobei die zwei Signalwerte als RLUm beziehungsweise RLUk gekennzeichnet werden, und Kennzeichnen einer Wachstumsrate von RLUm bis RLUk als A, wobei die Wachstumsrate A=(RLUm/RLUk-1)×100 % ist;
(a4) Plotten einer Standardkurve basierend auf einer Wachstumsrate A' von RLUm' bis RLUk' in beliebigen zwei Reaktionen in Schritt (a2) und Schritt (a3) in Bezug auf eine Reihe von Positivkontrollproben mit bekannten Konzentrationen, die das zu detektierende Zielmolekül enthalten; und
(a5) Vergleichen der in Schritt (a3) erhaltenen Wachstumsrate A mit der in Schritt (a4) erhaltenen Standardkurve, um eine Konzentration des zu detektierenden Zielmoleküls zu bestimmen,
wobei t, n, m und k alle natürliche Zahlen größer als 0 sind, und k<m≤n≤t, n≥2.

14. Verwendung des Kits nach Anspruch 13, wobei Schritt (a5) ist: Bestimmen basierend auf einem Wert von A, ob die Konzentration des zu detektierenden Zielmoleküls sich in einem steigenden Abschnitt oder einem sinkenden Abschnitt der Standardkurve befindet, und dann Berechnen der Konzentration des zu detektierenden Zielmoleküls, indem RLUm des zu detektierenden Zielmoleküls in eine entsprechende Standardkurve gesetzt wird.

15. Verwendung des Kits nach Anspruch 14, wobei Schritt (a5) ist: Vergleichen der Wachstumsrate A mit der Standardkurve, wobei
wenn sich die Wachstumsrate A in einem steigenden Abschnitt der Standardkurve befindet, Schritt (a2) gestoppt wird, und eine Konzentration des zu detektierenden Zielmoleküls durch direktes Setzen von RLUp, abgelesen zur p. Zeit, in die Standardkurve berechnet wird; und
wenn sich die Wachstumsrate A in einem sinkenden Abschnitt der Standardkurve befindet, der Schritt (a2) fortgesetzt wird, und eine Konzentration des zu detektierenden Zielmoleküls durch Setzen von RLUq der Reaktion, abgelesen zur q. Zeit, in die Standardkurve berechnet wird,
wobei p und q beide natürliche Zahlen größer als 0 sind, und p≤q≤n.

## Revendications

1. Procédé d'analyse immunitaire par chimioluminescence, ledit procédé comprenant les étapes suivantes :
(1) mélanger un échantillon à détecter suspecté de contenir une molécule cible à détecter à un réactif requis pour qu'une réaction immunitaire par chimiluminescence réagisse de manière à former un mélange à détecter ;
(2) amorcer le mélange à détecter t fois successivement pour générer une chimiluminescence, et enregistrer une valeur de signal de chimiluminescence n fois, une valeur de signal de chimiluminescence enregistrée au temps n étant marquée par RLUn ;
(3) sélectionner deux valeurs de signal quelconques parmi les valeurs de signal enregistrées n fois de la chimiluminescence, marquer les deux valeurs de signal respectivement par RLUm et RLUk, et marquer un taux de croissance à partir de RLUm à RLUk par A ;
(4) tracer une courbe standard sur la base d'un taux de croissance A' de RLUm' à RLUk' dans deux réactions quelconques à l'étape (2) et à l'étape (3) par rapport à une série de substances standard ayant des concentrations connues contenant la molécule cible à détecter ; et
(5) comparer le taux de croissance A obtenu à l'étape (3) à la courbe standard obtenue à l'étape (4) afin de déterminer une concentration de la molécule cible à détecter,
t, n, m et k étant tous des nombres naturels supérieurs à 0, et k < m ≤ n ≤ t, n ≥ 2.

2. Procédé selon la revendication 1, le taux de croissance A = (RLUm/RLUk-1) x 100 % ; et/ou n étant supérieur à 2.

3. Procédé selon la revendication 1, l'étape (5) étant une étape de comparaison du taux de croissance A obtenu à l'étape (3) à la courbe standard obtenue à l'étape (4),
lorsque le taux de croissance A est situé dans une section croissante de la courbe standard, l'étape (2) état arrêtée, et une concentration de la molécule cible à détecter étant calculée par placement direct de RLUp lu au temps p dans la courbe standard ; et
lorsque le taux de croissance A est situé dans une section décroissante de la courbe standard, l'étape (2) étant poursuivie et une concentration de la molécule cible à détecter étant calculée par placement de RLUq de la réaction lue au temps q dans la courbe standard,
p et q étant tous deux des nombres naturels supérieurs à 0, et p ≤ q ≤ n ;
de préférence, p étant 1 et q étant n.

4. Procédé selon la revendication 1, **caractérisé en ce que**, à l'étape (1), le réactif nécessaire à la réaction de chimiluminescence comprenant un réactif accepteur et un réactif donneur,
le réactif donneur comprenant un donneur, et le donneur étant capable de générer de l'oxygène singulet dans un état amorcé ; et
le réactif accepteur comprenant un accepteur, et l'accepteur étant capable de réagir avec l'oxygène singulet de manière à générer une valeur de signal de chimiluminescence détectable.

5. Procédé selon la revendication 1, **caractérisé en ce qu'**il comprend spécifiquement les étapes suivantes :
(a1) mélanger un échantillon à détecter suspecté de contenir un antigène (ou un anticorps) à détecter à un réactif accepteur et effectuer une première incubation, et mélanger un liquide mélangé obtenu à partir de la première incubation à un réactif donneur et effectuer une deuxième incubation de manière à former un mélange à détecter ;
(a2) amorcer le mélange à détecter t fois successivement avec irradiation d'un faisceau laser rouge de 600 à 700 nm afin générer une chimiluminescence, et enregistrer une valeur de signal de la chimiluminescence n fois avec une longueur d'onde de détection de 520 à 620 nm, une valeur de signal de la chimiluminescence enregistrée au temps n étant marquée par RLUn ;
(a3) sélectionner deux valeurs de signal quelconques à partir de n valeurs de signal enregistrées de la chimiluminescence, marquer les deux valeurs de signal respectivement par RLUm et RLUk, et marquer un taux de croissance de RLUm à RLUk par A, le taux de croissance A= (RLUm/RLUkl) × 100 % ;
(a4) tracer une courbe standard sur la base d'un taux de croissance A' de RLUm' à RLUk' dans deux réactions quelconques à l'étape (a2) et à l'étape (a3) par rapport à une série d'échantillons témoins positifs ayant des concentrations connues contenant la molécule cible à détecter ; et
(a5) comparer le taux de croissance A obtenu à l'étape (a3) à la courbe standard obtenue à l'étape (a4) afin de déterminer une concentration de la molécule cible à détecter,
t, n, m et k étant tous des nombres naturels supérieurs à 0 et k < m ≤ n ≤ t, n ≥ 2.

6. Procédé selon la revendication 5, l'étape (a5) étant une étape de détermination, sur la base d'une valeur de A, que la concentration de la molécule cible à détecter est située dans une section croissante ou une section décroissante de la courbe standard, et puis calculer la concentration de la molécule cible à détecter par placement de RLUm de la molécule cible à détecter dans une courbe standard correspondante.

7. Procédé selon la revendication 5, l'étape (a5) étant une étape de comparaison du taux de croissance A à la courbe standard,
lorsque le taux de croissance A est situé dans une section croissante de la courbe standard, l'étape (a2) étant arrêtée, et une concentration de la molécule cible à détecter étant calculée par placement direct de RLUp lu au temps p dans la courbe standard ; et
lorsque le taux de croissance A est situé dans une section décroissante de la courbe standard, l'étape (a2) étant poursuivie et une concentration de la molécule cible à détecter étant calculée par placement d RLUq de la réaction lue au temps q dans la courbe standard,
p et q étant tous deux des nombres naturels supérieurs à 0 et p ≤ q ≤ n.

8. Système utilisant le procédé d'analyse immunitaire par chimiluminescence, ledit système comprenant :
un dispositif de réaction, qui est configuré pour réaliser une réaction de chimiluminescence à l'intérieur ;
un dispositif d'amorçage et d'enregistrement, qui est configuré pour amorcer un mélange à détecter t fois successivement afin de générer une chimiluminescence, et enregistrer une valeur de signal de la chimiluminescence n fois, une valeur de signal de la chimiluminescence étant enregistrée au temps n étant marqué par RLUn ; et qui est configuré pour sélectionner deux valeurs de signal quelconques parmi les valeurs de signal enregistrées au temps n de la chimiluminescence, marquer les deux valeurs de signal respectivement par RLUm et RLUk, et marquer un taux de croissance de RLUm à RLUk par A ; et
un processeur, qui est configuré pour tracer une courbe standard sur la base d'un taux de croissance A' de RLUm' à RLUk' dans deux réactions quelconques par rapport à une série de substances standard ayant des concentrations connues contenant la molécule cible à détecter ; et qui est configuré pour comparer le taux de croissance A à la courbe standard afin de déterminer une concentration de la molécule cible à détecter,
t, n, m et k sont tous des nombres naturels supérieurs à 0, et k < m ≤ n≤t, n ≥ 2.

9. Système selon la revendication 8, un procédé d'utilisation du système comprenant les étapes suivantes :
(1) mélanger un échantillon suspecté de contenir une molécule cible à détecter à un réactif requis pour qu'une réaction de chimiluminescence réagisse de manière à former un mélange à détecter ;
(2) amorcer le mélange à détecter t fois successivement afin de générer une chimiluminescence, et enregistrer une valeur de signal de chimiluminescence n fois, une valeur de signal de chimiluminescence enregistrée à un temps n étant marquée par RLUn ;
(3) sélectionner deux valeurs de signal quelconques parmi les valeurs de signal de chimiluminescence enregistrées n fois, marquer les deux valeurs de signal respectivement par RLUm et RLUk, et marquer un taux de croissance de RLUm à RLUk par A ;
(4) tracer une courbe standard sur la base d'un taux de croissance A' de RLUm' à RLUk' dans deux réactions quelconques à l'étape (2) et à l'étape (3) par rapport à une série de substances standard ayant des concentrations connues contenant la molécule cible à détecter ; et
(5) comparer le taux de croissance A obtenu à l'étape (3) à la courbe standard obtenue à l'étape (4) afin de déterminer une concentration de la molécule cible à détecter,
t, n, m et k étant tous des nombres naturels supérieurs à 0 et k < m < n < t, n ≥ 2.

10. Système selon la revendication 8, le taux de croissance A = (RLUm/RLUk-1) x 100 % ; et/ou, n étant supérieur à 2.

11. Système selon la revendication 9, l'étape (5) étant une étape de comparaison du taux de croissance A obtenu à l'étape (3) à la courbe standard obtenue à l'étape (4),
lorsque le taux de croissance A est situé dans une section croissante de la courbe standard, l'étape (2) étant arrêtée et une concentration de la molécule cible à détecter étant calculée par placement direct de RLUp lu au temps p dans la courbe standard ; et
lorsque le taux de croissance A est situé dans une section décroissante de la courbe standard, l'étape (2) étant poursuivie et une concentration de la molécule cible à détecter étant calculée par placement de RLUq de la réaction lue au temps q dans la courbe standard,
t, n, m, k, p et q étant tous des nombres naturels supérieurs à 0, et k < m ≤ n ≤ t, n ≥ 2 ;
de préférence, p étant 1 et q étant n.

12. Utilisation d'un kit comprenant un réactif nécessaire à une analyse par chimiluminescence dans un procédé comprenant les étapes suivantes :
(1) mélanger un échantillon suspecté de contenir une molécule cible à détecter à un réactif requis pour qu'une réaction de chimiluminescence réagisse de manière à former un mélange à détecter ;
(2) amorcer le mélange à détecter t fois successivement afin de générer une chimioluminescence, et enregistrer une valeur de signal de chimioluminescence n fois, une valeur de signal de chimioluminescence enregistrée au temps n étant marquée par RLUn ;
(3) sélectionner deux valeurs de signal quelconques parmi les valeurs de signal enregistrées n fois de la chimiluminescence, marquer les deux valeurs de signal respectivement par RLUm et RLUk, et marquer un taux de croissance à partir de RLUm à RLUk par A ;
(4) tracer une courbe standard sur la base d'un taux de croissance A' de RLUm' à RLUk' dans deux réactions quelconques à l'étape (2) et à l'étape (3) par rapport à une série de substances standard ayant des concentrations connues contenant la molécule cible à détecter ; et
(5) comparer le taux de croissance A obtenu à l'étape (3) à la courbe standard obtenue à l'étape (4) afin de déterminer une concentration de la molécule cible à détecter,
t, n, m et k étant tous des nombres naturels supérieurs à 0, et k < m ≤ n ≤ t, n ≥ 2.

13. Utilisation du kit selon la revendication 12, le procédé d'utilisation du kit comprenant les étapes suivantes :
(a1) mélanger un échantillon à détecter suspecté de contenir un antigène (ou un anticorps) à détecter à un réactif accepteur et effectuer une première incubation, et mélanger un liquide mélangé obtenu à partir de la première incubation à un réactif donneur et effectuer une deuxième incubation de manière à former un mélange à détecter ;
(a2) amorcer le mélange à détecter t fois successivement avec irradiation d'un faisceau laser rouge de 600 à 700 nm afin générer une chimiluminescence, et enregistrer une valeur de signal de la chimiluminescence n fois avec une longueur d'onde de détection de 520 à 620 nm, une valeur de signal de la chimiluminescence enregistrée au temps n étant marquée par RLUn ;
(a3) sélectionner deux valeurs de signal quelconques à partir de n valeurs de signal enregistrées de la chimiluminescence, marquer les deux valeurs de signal respectivement par RLUm et RLUk, et marquer un taux de croissance de RLUm à RLUk par A, le taux de croissance A= (RLUm/RLUkl) × 100 % ;
(a4) tracer une courbe standard sur la base d'un taux de croissance A' de RLUm' à RLUk' dans deux réactions quelconques à l'étape (a2) et à l'étape (a3) par rapport à une série d'échantillons témoins positifs ayant des concentrations connues contenant la molécule cible à détecter ; et
(a5) comparer le taux de croissance A obtenu à l'étape (a3) à la courbe standard obtenue à l'étape (a4) afin de déterminer une concentration de la molécule cible à détecter,
t, n, m et k étant tous des nombres naturels supérieurs à 0 et k < m ≤ n ≤ t, n ≥ 2.

14. Utilisation du kit selon la revendication 13, l'étape (a5) étant une étape de détermination, sur la base d'une valeur de A, que la concentration de la molécule cible à détecter est située dans une section croissante ou une section décroissante de la courbe standard, puis calculer la concentration de la molécule cible à détecter par placement de RLUm de la molécule cible à détecter dans une courbe standard correspondante.

15. Utilisation du kit selon la revendication 14, l'étape (a5) étant une étape de compasraison du taux de croissance A la courbe standard,
lorsque le taux de croissance A est situé dans une section croissante de la courbe standard, l'étape (a2) étant arrêtée, et une concentration de la molécule cible à détecter étant calculée par placement direct de RLUp lu au temps p dans la courbe standard ; et
lorsque le taux de croissance A est situé dans une section décroissante de la courbe standard, l'étape (a2) étant poursuivie et une concentration de la molécule cible à détecter étant calculée par placement de RLUq de la réaction lue au temps q dans la courbe standard,
p et q étant tous deux des nombres naturels supérieurs à 0 et p ≤ q ≤ n.
